Europäisches Patentamt

European Patent Office

Office européen des brevets

(19)

(11) Numéro de publication: **0 172 045**
**B1**

(12) **FASCICULE DE BREVET EUROPEEN**

(45) Date de publication du fascicule du brevet:
08.02.89

(51) Int. Cl.⁴: **C 07 K 15/14**, A 61 K 37/02 //
A61K39/395

(21) Numéro de dépôt: 85401201.0

(22) Date de dépôt: 18.06.85

(54) Glycoprotéines antitumorales, modifiées sur leurs motifs glucidiques, procédé d'obtention.

(30) Priorité: 20.06.84 FR 8409703
13.02.85 FR 8502067

(43) Date de publication de la demande:
19.02.86 Bulletin 86/8

(45) Mention de la délivrance du brevet:
08.02.89 Bulletin 89/6

(84) Etats contractants désignés:
AT BE CH DE FR GB IT LI LU NL SE

(56) Documents cités:
EP-A- 0 074 279
FR-A- 2 312 259

BIOLOGICAL ABSTRACTS, vol. 79, 1985, résumé no.
95713, Philadelphia, US; P.E. THORPE et al.:
"Modification of the cabohydrate in ricin with
metaperiodate-cyanoborohydride mixtures effects on
toxicity and in vivo distribution" & EUR. J. BIOCHEM.
(WEST GERMANY), vol. 147, no. 1, 197-206, 1985

(73) Titulaire: SANOFI, 40, Avenue George V, F-75008 Paris
(FR)

(72) Inventeur: Jansen, Franz K., Chemin des Fresquets
Assas, F-34160 Castries (FR)
Inventeur: Gros, Pierre, 18 rue des Muriers, F-34100 -
Montpellier (FR)

(74) Mandataire: Gillard, Marie-Louise et al, Cabinet Beau de
Loménie 55, Rue d'Amsterdam, F-75008 Paris (FR)

ACTORUM AG

## Description

La présente invention concerne de nouvelles glycoprotéines antitumorales et inactivant les ribosomes, dont les motifs glucidiques sont modifiés par oxydation par l'ion périodate, lesdites glycoprotéines étant différentes de la ricine entière.

Plus particulièrement, la présente invention se réfère à de nouvelles glycoprotéines inactivant les ribosomes et à longue durée d'action, lesdites glycoprotéines étant différentes de la ricine entière.

Le terme «glycoprotéine inactivant les ribosomes», tel qu'utilisé dans la présente description ainsi que dans les revendications, désigne toute substance portant des motifs saccharidiques appartenant à la classe des macromolécules inactivant les ribosomes et, par conséquent inhibant la synthèse protéique de cellules eucaryotes, ainsi que tout fragment de ladite substance possédant la même propriété inactivante, ladite glycoprotéine inactivant les ribosomes pouvant être d'origine naturelle ou biosynthéthique provenant d'une cellule dont le patrimoine génétique a été modifié dans ce but, ladite glycoprotéine inactivant les ribosomes pouvant également être modifiée sur les groupes fonctionnels de ses acides aminés de façon à pouvoir être aisément couplée à un anticorps.

Dans la description, l'expression «glycoprotéine inactivant les ribosomes» sera sybolisée GPIR.

Dans la description, le terme «periodate» désigne l'ion $IO^{-4}$ que l'on trouve également indiqué dans la littérature sous le nom de «métaperiodate».

Les glycoprotéines inactivant les ribosomes (GPIR) sont notamment utiles comme intermédiaires dans la préparation d'immunotoxines, par couplage avec des anticorps.

Dans le brevet US 4 340 535 et dans les demandes de brevets français n° 2 504 010 et n° 2 516 794 est décrite la préparation de produits anti-canćreux dits conjugués obtenus par couplage, par liaison covalente, de la chaîne A de ricine avec des anticorps dirigés contre des antigènes portés par la cellule à détruire. Les produits de ce type ont été désignés et sont désignés dans la présente demande sous le nom générique d'Immunotoxines.

On connaît aussi des conjugués analogues aux immunotoxines à chaîne A de ricine précédemment décrites, ayant aussi vocation de médicaments anti-cancéreux et résultant du couplage par liaison covalente d'anticorps ou fragments d'anticorps à d'autres glycoprotéines inactivant les ribosomes telles que notamment la gélonine extraite de Gelonium multiflorum (Eur. J. Biochem., 1981, 116, 447–454, Cancer Res., 1984, 44, 129–133) ou l'inhibiteur extraît de Momordica charantia (MOM) (brevet US 4 368 149).

Ces glycoprotéines inactivant les ribosomes (GPIR) qui ont des propriétés semblables à celles de la chaîne A de ricine sont des substances de poids moléculaire d'un ordre de grandeur de 20 000 et 30 000 (Cancer Survey, 1982, 1, 489 à 520).

On sait également que l'activité cytotoxique de ces immunotoxines peut être potentialisée par diverses substances adjuvantes telles que les sels d'ammonium, diverses amines, ou certains ionophores carboxyliques, tels que la monensine, la nigéricine.

Toutefois, les effets thérapeutiques des immunotoxines, activées ou non, ne peuvent se manifester pleinement que dans la mesure où l'immunotoxine peut, par sa partie anticorps, se localiser in vivo, sous forme active, sur la cible cellulaire à détruire (condition sine qua non à toute expression d'activité des immunotoxines). La capacité de l'immunotoxine à se localiser sur la cible dépend en tout premier lieu de l'aptitude de l'immunotoxine à demeurer dans la circulation sanguine et les fluides extra-cellulaires sous forme active pendant des temps suffisants pour qu'elle atteigne sa cible cellulaire et à des concentrations suffisamment fortes pour que le taux d'occupation des sites antigéniques correspondants soit élevé.

De nombreuses études ont permis d'établir la cinétique d'élimination plasmatique des immunotoxines après injection intra-veineuse dans différents modèles animaux. Il est apparu qu'après l'injection, le taux plasmatique d'immunotoxine biologiquement active décroît très rapidement et de façon très importante. Ainsi, typiquement, chez le lapin, dans un modèle utilisant une immunotoxine construite en couplant à l'aide d'un bras à pont disulfure, la chaîne A de ricine à un anticorps monoclonal dirigé contre l'antigène T65 des lymphocytes T humains (anticorps T101), il apparaît que 97 % de l'immunotoxine présente dans le sang circulant au temps 0 de l'injection disparaissent en 30 minutes et 99,9 % en 17 heures. Cette rapide disparition de l'immunotoxine est bien évidemment préjudiciable à l'expression de sa capacité cytotoxique complète, en empêchant que l'immunotoxine ne sature de façon durable une proportion élevée des antigènes cibles portés par les cellules à détruire. En outre, la comparaison des cinétiques d'élimination plasmatique des immunotoxines avec celles des anticorps non conjugués correspondants montre, qu'à l'inverse, les anticorps – comme cela est bien connu – se maintiennent dans le plasma à un haut niveau pendant des temps relativement longs. Or il existe toujours, même dans les préparations d'immunotoxines les plus purifiées, un certain taux résiduel d'anticorps non conjugués. Par le jeu des vitesses différentielles d'élimination des immunotoxines et des anticorps, progressivement les anticorps non conjugués, initialement très minoritaires, deviennent majoritaires au bout de quelques heures et donc, progressivement, ces anticorps deviennent par compétition de puissants antagonistes pour la fixation des immunotoxines sur leurs cibles.

Il apparaît donc clairement l'intérêt d'accroître la persistance plasmatique des immunotoxines, sous forme active, afin d'augmenter à la fois la durée et le taux d'occupation des antigènes-cibles et par conséquent d'améliorer les effets thérapeutiques des immunotoxines.

Par ailleurs, des expériences de localisation in vivo de l'immunotoxine à chaîne A de ricine, radiomarquée puis injectée chez des animaux sans cible spécifique ont montré que, dans les premières minutes après l'injection, le conjugué se localise préférentiellement dans le foie. Il en est de même pour la chaîne A de ricine qui présente le même devenir lorsqu'elle est injectée sous forme non couplée. Cela suggère fortement que l'immunotoxine se fixe dans le foie l'intermédiaire de la sous-unité cytotoxique qu'elle contient. Il est connu que la chaîne A de ricine est une glycoprotéine dont les groupements polyosidiques comprennent notamment des résidus de mannose et de N-acétylglucosamine, certains résidus de mannose étant en positions terminales (Agri. Biol. Chem., 1978, 42, 501). Il a été également établi l'existence au niveau du foie de récepteurs capables de reconnaître des glycoprotéines ayant de tels résidus de mannose terminaux. Il a été aussi montré que les glycoprotéines reconnues par ces récepteurs – présents essentiellement sur les cellules de Kupffer – sont rapidement éliminées de la circulation sanguine par fixation sur ces cellules qui les métabolisent. Ceci est bien documenté notamment dans le cas de la bêta-glucuronidase, ainsi que dans le cas de la ribonucléase B (Arch. Biochem. Biophys., 1978, 188, 418; Advances in Enzymology, Meister A. Ed., New York, 1974; Pediat. Res., 1977, 11, 816).

De l'ensemble de ces données, il apparaît que la rapide élimination des immunotoxines à chaîne A de ricine peut s'expliquer par la reconnaissance des résidus mannose de la chaîne A de ricine par les cellules hepatiques et en particulier les cellules de Kupffer.

Les études de cinétique d'élimination plasmatique d'autres GPIR, par exemple la gélonine ou le MOM, après injection intra-veineuse chez l'animal, ont montré que, comme dans le cas de la chaîne A de ricine, le taux plasmatique de GPIR décroît très rapidement et de façon très importante après l'injection. Ainsi typiquement chez le lapin après injection de gélonine purifiée selon la méthode décrite (J. Biol. Chem., 1980, 255, 6947–6953) il apparaît que 93% de la gélonine présente dans le sang circulant au temps 0 de l'injection, disparaissent en 1 h et 99,99% en 24 heures.

Il est connu que par oxydation par les ions periodate des structures osidiques, y compris celles qui sont contenues dans les glycoprotéines, on provoque la rupture de la chaîne carbonée chaque fois que deux atomes de carbone adjacents portent des hydroxyles primaires ou secondaires. Si les deux hydroxyles contigus sont secondaires, comme cela est généralement le cas dans les oses cycliques présents dans les GPIR, l'oxydation conduit à deux fonctions aldéhyde sur les carbones entre lesquels la rupture a eu lieu.

On a maintenant trouvé que lorsqu'on modifie les motifs glucidiques d'une glycoprotéine antitumorale par oxydation avec les ions periodate, l'activité biologique de celle-ci reste substantiellement inchangée.

On a également trouvé, d'une façon absolument inattendue, que, si on modifie les motifs glucidiques d'une glycoprotéine inactivant les ribosomes par une oxydation par les ions periodate, on obtient une nouvelle glycoprotéine inactivant les ribosomes ayant la double propriété de conserver ses activités biologiques et d'être éliminée très lentement de la circulation sanguine in vivo.

Une étude biochimique approfondie des GPIR natives et oxydées a permis de mettre en évidence que l'oxydation periodique des GPIR concerne exclusivement la partie osidique des GPIR et est sans action sur la séquence des acides aminés qui en constituent la partie peptidique.

Ces nouvelles glycoprotéines inactivant les ribosomes et à longue durée d'action, sont ci-après symbolisées GPIR-La.

On a enfin trouvé que lorsque ces nouvelles glycoprotéines inactivant les ribosomes et à longue durée d'action sont couplées à des anticorps, les conjugués obtenus conservent les propriétés biologiques connues pour des immunotoxines et présentent une cinétique d'élimination plasmatique lente.

On notera que dans l'art antérieur, l'oxydation periodique a été proposée pour la fabrication de conjugués constitués d'un anticorps ou d'un fragment d'anticorps et d'une substance antitumorale. Dans ce cas, les groupes aldéhydiques obtenus par oxydation periodique de la substance anti-tumorale sont utilisés pour réaliser le conjugué souhaité. Ces groupes aldéhydiques sont donc introduits temporairement pour permettre la conjugaison anticorps-substance antitumorale. A cet effet, on peut citer les brevets EP 74 279 et le brevet FR 2 312 259 dans lesquels les glycoprotéines mises en œuvre dans la présente demande ne sont pas citées.

Au contraire, dans la présente demande, les groupes aldéhydiques introduits sur la glycoprotéine par l'oxydation periodique ne sont pas impliqués dans la formation des immunotoxines définies ci-dessus.

La présente invention a donc pour objet, à titre de produits nouveaux, des glycoprotéines antitumorales modifiées dans leur structure, dont les motifs glucidiques sont modifiés par oxydation par l'ion periodate.

La présente invention a plus particulièrement pour objet des glycoprotéines inactivant les ribosomes, dont les motifs glucidiques sont modifiés par oxydation par l'ion periodate, ayant la même activité et une demi-vie plus longue que celle de la glycoprotéine non modifiée.

De façon préférentielle, l'invention a pour objet des glycoprotéines inactivant les ribosomes et à longue durée d'action, caractérisées en ce qu'elles sont obtenues par traitement d'une glycoprotéine inactivant les ribosomes, dont les groupes thiol sont éventuellement protégés, avec une solution aqueuse d'un periodate alcalin pendant une période de temps de 0,2 à 24 heures à la température de 0 à 15°C et à l'abri de la lumière, par déblocage éventuel des groupes thiol et isolement du produit final selon des méthodes connues.

Toute glycoprotéine antitumorale peut être modifiée sur ses motifs glucidiques par action de l'ion periodate selon les méthodes connues.

Des glycoprotéines inactivant les ribosomes utilisées comme produits de départ préférentiels pour l'oxydation par les ions periodate selon l'invention sont toutes les GPIR, comme la chaîne A de ricine, qui sont par elles-mêmes très faiblement cytotoxiques car elles ne peuvent se fixer sur les cellules, mais qui, par contre, arpès couplage à un anticorps reconnaissant des cellules particulières, deviennent hautement cytotoxiques pour ces cellules une fois que l'anticorps a reconnu sa cible.

Les composés de départ représentatifs sont la chaîne A de ricine, la gélonine et la substance extraite de Momordica charantia (MOM), telles qu'elles sont obtenues par la voie extractive.

D'autres GPIR utiles comme produits de départ pour l'oxydation par les ions périodate sont les suivants:

| | |
|---|---|
| Dianthin 30 | de Dianthus caryophyllus |
| Dianthin 32 | de Dianthus caryophyllus |
| Agrostin A | de Agrostemma githago |
| Agrostin B | de Agrostemma githago |
| Agrostin C | de Agrostemma githago |
| HCl | de Hura crepitans |
| Asparagus officinalis inhibitor | de asparagus officinalis |

Les mêmes substances, produites par la voie biosynthétique par des cellules dont le patrimoine génétique a été modifié dans ce but sont des composés également convenables.

Des fragments des GPIR ci-dessus, à condition que de tels fragments retiennent tout ou partie de la propriété d'inactivation des ribosomes qui caractérise la GPIR dont ils sont issus, peuvent également être utilisés comme produits de départ.

La chaîne A de ricine native, dont au moins l'un des groupements thiol est protégé, est un composé de départ préférentiel.

Des études récentes ont mis en évidence que la chaîne A de ricine comprenait 2 constituants désignés par A1 et A2 qui diffèrent notamment par leurs motifs polysaccharidiques. Les expériences réalisées sur les 2 constituants de la chaîne A ont permis de mettre en évidence que l'oxydation periodique d'effectuait de façon similaire sur les chaînes A1 et A2 et conférait à ces 2 constituants des propriétés identiques d'amélioration de la pharmacocinétique.

L'obtention de la chaîne A de la ricine pure est décrite dans le brevet US 4 340 535. La gélonine et le MOM sont également décrits dans la littérature.

La protection des groupes thiol des GPIR de départ est souhaitable lorsque lesdits groupes thiol sont ceux qui seront utilisés pour le couplage avec l'anticorps. Si pour le couplage on utilise d'autres groupes fonctionnels, par exemple l'hydroxyle phénolique des tyrosines ou des groupes aminés ou des groupes carboxyliques de la GPIR, la protection peut ou non être effectuée.

Le blocage est effectué par réaction avec un agent susceptible de substituer les fonctions SH à l'aide d'un radical qui peut être ensuite éliminé par réduction ou échange thiol/disulfure, par exemple par l'acide dinitro-2,2' dithio 5,5' dibenzoique (DTNB) ou bien par l'acide (pyridyl-2 disulfanyl)-3 propionique ou encore par le dipyridyl-2,2' disulfure ou le dipyridyl-4,4' disulfure. En l'absence d'un tel traitement, les thiols libres peuvent disparaître pendant la réaction d'oxydation et dans ce cas ne peuvent être totalement régénérés. L'excès de l'agent bloquant est éliminé par dialyse ou tout autre traitement approprié.

La réaction d'oxydation periodique est effectuée à un pH acide, compris entre 3 et 7, de préférence entre 5 et 6,5.

Le periodate est utilisé en excès; plus particulièrement, la concentration en periodate alcalin est supérieure à la concentration des diols vicinaux susceptibles d'être oxydés: des concentrations de 10 à 50 mM en periodate de sodium pour des concentrations de 1 à 10 mg/ml de sous-unité cytotoxique sont convenables. La durée du traitement, réalisé à une température comprise entre 0 et 15°C et de préférence entre 1 et 5°C et à l'obscurité est comprise entre 0,2 et 24 heures.

La réaction est arrêtée par addition d'un réactif qui consomme le periodate restant, par exemple un excès d'éthylène-glycol et les sous-produits sont éliminés par dialyse ou par tout autre traitement approprié. Le produit obtenu à la fin de la réaction est isolé selon les techniques conventionnelles.

Si les groupes thiol du produit de départ étaient bloqués, le déblocage est effectué selon les méthodes connues, par exemple par action d'un agent réducteur apte à libérer la fonction thiol préalablement bloquée tel que le mercapto-2 éthanol, ce qui conduit à l'obtention de la nouvelle glycoprotéine inactivant les ribosomes et à longue durée d'action prête à être utilisée, par exemple pour le couplage avec un anticorps pour obtenir une immunotoxine.

Dans le cas de la chaîne A de ricine, la nouvelle molécule ainsi obtenue (symbolisée A–1a) possède les propriétés principales suivantes:

– un poids moléculaire non significativement différent de celui de la chaîne A native. Ce procédé de modification ne fait apparaître de façon visible par électrophorèse en gradient de polyacrylamide des polymères de la protéine, qu'en très faible quantité et aucuns produits de dégradation,

– un taux de groupements thiol libres supérieur à 0,7 par mole,

– une immunoréactivité envers des anticorps de lapin anti-chaîne A de ricine que l'on ne peut distinguer de celle de la chaîne A native,

– une activité inhibitrice de la synthèse protéique dans un modèle acellulaire supérieure à 50% de celle provoquée par une égale quanitité de chaîne A native,

– enfin, après administration intraveineuse unique chez le lapin à une dose voisine de 0,4 mg/kg de poids corporel, le taux plasmatique de la chaîne A à longue durée d'action (A–L a) au temps 23 heures après l'injection est supérieur à 10% du taux présent au temps zéro (contre 0,015% pour la chaîne A native à ce même temps), soit un accroissement du taux plasmatique par un facteur largement supérieur à 500.

De même dans le cas de la gélonine, la molécule obtenue par oxydation periodique possède les propriétés principales suivantes:

– un poids moléculaire non significativement différent de celui de la gélonine native,

– une immunoréactivité envers anticorps de lapin anti-gélonine que l'on ne peut distinguer de celle de la gélonine native,

– enfin, après administration intraveineuse unique chez le lapin à une dose voisine de 0,3 mg/kg de poids corporel, le taux plasmatique de la gélonine modifiée, au temps 24 heures après injection est supérieur à 3% du taux présent au temps zéro (contre 0,01% pour la gélonine native à ce même temps, soit un accroissement du taux plasmatique par un facteur supérieur à 200).

La préparation des conjugués ou immunotoxines à partir des ghlycoprotéines inactivant les ribosomes et à longue durée d'action est réalisée par n'importe quel procédé convenablement choisi dans la gamme de ceux qui sont décrits dans le brevet US 4 340 535. Si la sous-inité cytotoxique choisie présente naturellement au moins un thiol la rendant apte au couplage, cette fonction sera préférentiellement mise on œuvre par réaction avec l'anticorps ou fragment d'anticorps porteur d'un groupement disulfure activé. Si la sous-unité cytotoxique choisie ne possède pas naturellement de groupe thiol la rendant apte au couplage on pourra préférentiellement introduire artificiellement sur la dite sous-unité, au moins un groupement fonctionnel porteur d'un thiol libre, selon tout procédé connu, et poursuivre le couplage comme indiqué ci-dessus. L'introduction du dit groupement fonctionnel peut avoir lieu soit avant l'étape d'oxydation par les ions periodate mais il sera alors nécessaire que le radical thiol soit bloqué pendant l'étape d'oxydation puis débloqué après cette étape, soit après l'étape d'oxydation.

On obtient ainsi des immunotoxines modifiées qui ont acquis un caractère nouveau quant à leurs propriétés pharmacocinétiques. Plus particulièrement, par modification appropriée de la sous-unité cytotoxique on a pu ajouter aux propriétés de cytotoxicité spécifique des immonotoxines, sans qu'il leur soit porté atteinte, une nouvelle propriété tout aussi intrinsèque: la capacité à manifester une lente cinétique d'élimination plasmatique.

Les exemples suivants permettent de mieux comprendre l'invention sans en limiter la portée.

Exemple 1:
Oxydation de la chaîne A méthylée dont les SH sont bloqués par la N-éthylmaléimide.

I – Préparation de la chaîne A de ricine correctement fonctionnalisée:
1) Héxaméthylation de la chaîne A:

La réaction de méthylation s'effectue à 0°C sous agitation dans du tampon borate 0,2 M pH 10 selon la méthode de Means et Feeney (Biochemistry 7, 2192 (1968). A 35 ml de chaîne A (3 mg/ml) on ajoute successivement 20 mg de borohydrure trité (à 9,5 mCi/mmole) puis 350 microlitres de formaldéhyde à 6% (en 5 additions de 70 microlitres chacune échelonnées sur un délai de 30 min).

On élimine l'excès de réactif par dialyse en continu contre du tampon phosphate 125 mM pH 7 (40 l à 300 ml/h). Après dialyse la solution protéique est centrifugée. On recueille 36,5 ml de chaîne A hexaméthylée à 2,6 mg/ml.

2) Blocage au N-éthyl maléimide:
On procède au blocage de SH naturel de la chaîne A héxaméthylée selon la méthode décrite dans Methods in Enzymology 11, 541, (1967). Pour cela, la chaîne A de ricine obtenue à l'étape précédente est incubée pendant 2 heures à 30°C en présence de 20 équivalents de N-éthylmaléimide par mole de chaîne A. On élimine l'excès de réactif par dialyse en continu contre du tampon phosphate 125 mM pH 7 renouvelé pendant 20 heures à raison de 500 ml/heure. Après concentration, on obtient ainsi 13 ml d'une solution de chaîne A de ricine à 7 mg/ml, ne présentant plus de groupements thiol dosables par le réactif d'ELLMAN. Le produit ainsi obtenu est désigné ultérieurement sous l'appellation chaîne A (NEM) hexaméthylée.

3) Oxydation periodique:
6 ml de la solution de chaîne A (NEM) hexaméthylée précédemment obtenue, sont traités par NaIO4 (12,8 mg) en 40 minutes à l'obscurité, à pH 4,5 et à 0°C. La réaction est arrêtée par addition de 600 microlitres d'éthylène glycol et le milieu réactionnel est dialysé en continu contre du tampon carbonate 0,1 M, pH 10 (20 h à 500 ml/h).

II – Activité enzymatique de la chaîne A à longue durée d'action, mesurée sur un modèle acellulaire.

La propriété biologique fondamentale de la chaîne A de ricine est d'inhiber la synthèse protéique cellulaire par altération de la sous-unité ribosomale 60 S.

Le protocole in vitro utilisé, comporte l'emploi de fractions subcellulaires de foie de rat convenablement complémentées et capables à incorporer la 14C-phénylalanine en présence d'un ARN messager artificiel, l'acide polyuridylique.

Le mode opératoire employé pour préparer les fractions subcellulaires et pour mesurer l'incorporation de 14C-phénylalanine est une adaptation de la méthode décrite dans Biochemica Biophysica Acta 1973, 312, 608–615, mettant en œuvre à la fois une fraction microsomale et une fraction de cytosol des hépatocytes de rat. L'échantillon contenant la chaîne A est introduit sous la forme d'une solution convenablement diluée dans un tampon Tris HCl 50 mM, pH 7,6 contenant du mercapto-2 éthanol à 0,2% et de la sérum albumine bovine à 15 microgrammes-ml.

A partir des données de comptage, on calcule par rapport à un milieu témoin sans inhibiteur le pourcentage d'inhibition de l'incorporation de 14C-phénylalanine dans les protéines par chaque milieu réactionnel contenant de la chaîne A de ricine.

L'activité inhibitrice a été déterminée. On observe une CI50 égale à $2,7.10^{-10}$ mole/1 pour la chaîne A oxydée. La CI50 de la chaîne A témoin de l'expérience est de $1,03.10^{-10}$ mole/1: la modification n'entraîne donc pas de perte d'activité de la chaîne A.

Exemple 2:

Cet exemple démontre, après injection intraveineuse chez l'animal, l'élimination lente de la chaîne A de ricine modifiée par le periodate de sodium.

I – Modification de la chaîne A de ricine par le periodate de sodium.

1) Blocage du SH naturel par le DTNB.

La chaîne A de ricine a été préparée et purifiée ainsi qu'il a été indiqué dans le brevet US 4 340 535. A 10 ml d'une solution de chaîne A de ricine à 5,6 mg/ml (à 0,84 groupement thiol par chaîne A) dans le tampon PBS (tampon phosphate 20 mM, NaCl 150 mM, pH7) on ajoute 20 équivalents d'une solution d'acide dinitro-2,2' dithio-5,5' dibenzoique (DTNB) soit 385 microlitres d'une solution 0,1 M de DTNB dans un tampon phosphate 125 mM pH 7 (cette solution est amenée à pH 7 à l'aide d'hydroxyde de sodium). On laisse l'incubation se poursuivre pendant 20 minutes à 20°C. On dialyse ensuite la solution contre du tampon PBS à 4°C, on obtient ainsi 53 mg de chaîne A bloquée sur la fonction thiol, en solution à 5 mg/ml.

2) Oxydation periodique de la chaîne A bloquée:

A 6 ml de solution à 5 mg/ml de chaîne A bloquée, amenée à pH 6 à l'aide d'acide acétique 1 M, on ajoute 120 microlitres d'une solution de periodate de sodium 0,5 M dans l'eau. On laisse l'incubation se poursuivre pendant 16 heures à 4°C dans l'obscurité. La réaction d'oxydation est arrêtée par addition de 620 microlitres d'une solution aqueuse d'éthylène-glycol 1 M. Après 15 minutes d'incubation à 20°C, le milieu réactionnel est dialysé à 4°C contre du tampon PBS. L'oxydation periodique fait apparaître un léger précipité protéique qui est éliminé par centrifugation à 10 000 x g pendant 30 minutes. On obtient ainsi 24 mg de chaîne A bloquée et oxydée à une concentration de 3,4 mg/ml.

3) Déblocage des groupements thiol:

A 6 ml de chaîne A bloquée et oxydée à 3,4 mg/ml dans le tampon PBS on ajoute comme agent réducteur du mercapto-2 éthanol à la concentration finale de 1%. On laisse l'incubation se poursuivre pendant 1 heure à 20°C. On dialyse ensuite la solution contre le tampon PBS à 4°C. On obtient ainsi 19 mg de chaîne A oxydée à une concentration de 2,8 mg/ml.

En utilisant la technique au DTNB (Methods in Enzymology, 1972, 25, 457 (Academic Press) on détermine que la chaîne A modifiée obtenue présente 0,70 groupement thiol libre par mole. Le poids moléculaire de la chaîne A modifiée est de 30 000 +/- 3000, déterminé par électrophorèse en gradient de polyacrylamide en présence de dodecyl-sulfate de soldium.

La préparation de chaîne A dont les motifs polysaccharidiques ont été oxydés, obtenue précédemment, a été étudiée en ce qui concerne ses activités enzymatiques d'inhibition de la synthèse protéique et ses propriétés pharmacocinétiques.

II – Activité enzymatique de la chaîne A à longue durée d'action, mesurée sur un modèle acellulaire.

L'activité inhibitrice a été déterminée selon la technique décrite à l'exemple 1. On observe une CI50 égale à $3.10^{-10}$ mole/l pour la chaîne A oxydée. La CI50 de la chaîne A témoin de l'expérience est de $1,2.10^{-10}$ mole/l: la modification n'entraîne donc pas de perte d'activité de la chaîne A.

III – Propriétés pharmacocinétiques de la chaîne A à longue durée d'action (A–La).

La chaîne A est administrée chez le lapin par injection unique dans une veine de l'oreille. La quantité de chaîne A injectée correspond à 0,415 mg/kg. Les échantillons de sang sont prélevés au cours du temps sur héparine. Les plasmas sont analysés à l'aide d'un test immunoradiométrique ci-après désigné par l'abréviation IRM-1.

Cette technique a l'avantage de doser la chaîne A sans modification de celle-ci. Ce dosage est réalisé dans des plaques à microtitrations (par exemple: «NUNC-TSP screening system» Poly Labo Block France) dont le couvercle est muni de pointes hyperabsorbantes qui plongent dans les puits du socle. Ces pointes constituent les phases solides. Des anticorps de brebis anti-chaîne A de ricine (ci-après désignés sous l'abréviation AcI), purifiés par chromatographie d'affinité, sont absorbés sur les phases solides. Pour cela 200 microlitres d'une solution d'AcI à 10 microgrammes/ ml dans le tampon PBS sont distribués dans les puits. Les pointes sont mises en contact d'abord avec la solution d'AcI pendant 24 h à 4°C puis avec du sérum de veau fœtal pendant 3 h à 20°C pour saturer tous les sites de fixation. L'immunoabsorbant saturé est alors mis en contact pendant 3 h à 20°C avec les échantillons plasmatiques à doser à différentes dilutions ou avec des solutions de chaîne A de concentrations connues pour l'établissement de la courbe d'étalonnage. Un lavage est effectué par un tampon PBS, puis l'immunoabsorbant est mis en contact pendant 2 h à 20°C avec les anticorps de brebis anti-chaîne A de ricine purifiés par chromatographie d'affinité et radiomarqués (ci-après désignés par l'abréviation Ac2). Le radiomarquage de l'Ac2 est réalisé avec l'iode 125 en présence de chloramine T selon la méthode de Greenwood et Hunter (Biochem J., 1963, 89, 114); l'activité spécifique des Ac2 radiomarqués est de 5 à 10 microCuries-microgramme. $10^6$ cpm d'Ac2 radiomarqués sont introduits sous 200 microlitres dans un tampon PBS, contenant 0,1% de sérum albumine bovine. Après lavage, dans le tampon PBS, les pointes sont détachées et la quantité d'Ac2 lié est mesurée par comptage de la radioactivité. La mesure de la concentration en chaîne A dans les échantillons à doser se fait par référene à la courbe d'étalonnage réalisée avec la chaîne A introduite à différentes concentrations connues. Lorsque de la chaîne A à longue durée d'action est injectée chez l'animal, cette même chaîne A à longue durée d'action est utilisée pour l'établissement de la courbe d'étalonnage correspondante.

Les valeurs de concentration en chaîne A dans le plasma sanguin mesuré par cette technique sont reproductibles et fiables. Le seuil de détection est de 1 nanogramme/ml. L'étude de la reproductibilité intra- et inter-essais donne des coefficients de variation inférieurs à 10% pour les valeurs de concentration comprises dans la gamme de 1 à 200 nanogrammes/ml.

Les résultats de ces expériences sont représentés sous la forme de courbes présentant en abscisses le temps, exprimé en heure, et en ordonnées, sur échelle logarithmique, la concentration plasmatique du produit mesuré, rapportée en pourcent de la concentration plasmatique théorique au temps zéro. Cette valeur appelée «concentration plasmatique relative» (CPR) est calculée à l'aide de l'expression suivante:

$$CPR = \frac{\text{Concentration mesurée au temps t}}{\text{quantité injectée/volume plasmatique}} \times 100$$

Le volume plasmatique est considéré égal à 36 ml/kg de poids corporel de l'animal.

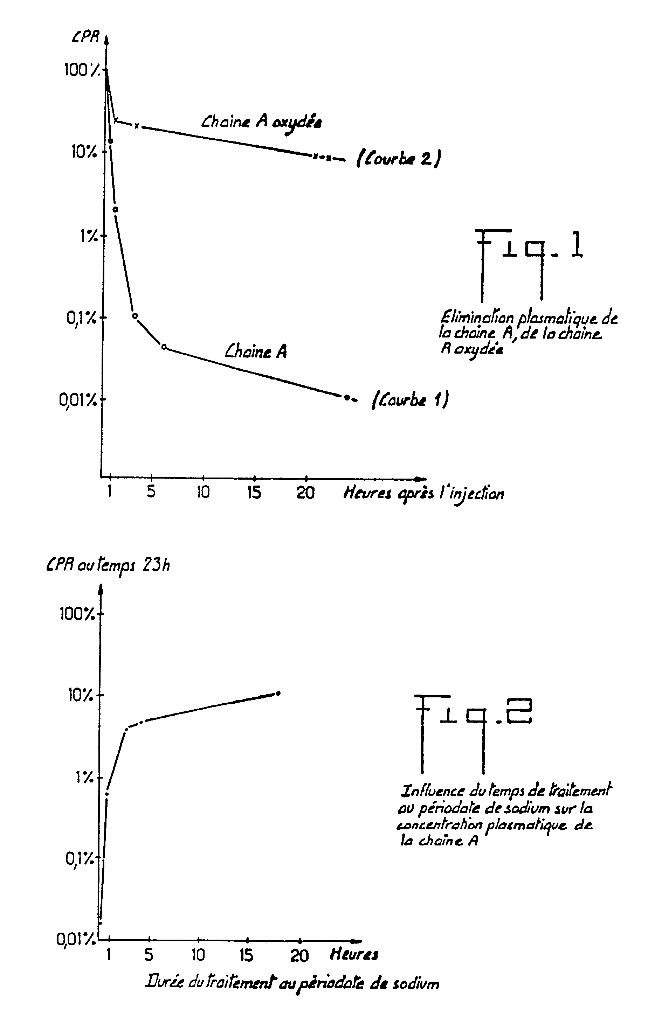

La figure 1 montre la courbe d'élimination plasmatique, en fonction du temps, de la chaîne A de ricine native injectée par voie intra-veineuse. Cette courbe (courbe 1) présente deux phases: la première phase, le produit disparaît très rapidement de la circulation puisque trois heures après l'injection il ne reste plus dans le plasma que 0,1% de la dose administrée. Dans la deuxième phase, la décroissance est plus lente.

Lorsque la chaîne A a été oxydée sur ses motifs polysaccharidiques, le profil d'élimination est profondément modifié: la première phase d'élimination – responsable de la disparition de la plus grande partie du produit – est pratiquement abolie, ce qui conduit à un accroissement considérable des taux plasmatiques de chaîne A. Vingt heures après l'injection, la concentration de la chaîne A oxydée est 600 fois supérieure à ce qu'elle est quand la chaîne A n'est pas modifiée (courbe 2).

Exemple 3:
Cet exemple démontre l'effet de l'oxydation periodique sur les propriétés pharmacocinétiques de la chaîne A bloquée au NEM.

1) – Modification de la chaîne A de ricine.
a) Blocage du SH naturel par la N-éthylmaléimide.
A 40 ml de solution aqueuse de chaîne A de ricine à 8 mg/ml (soit 4,1 micromoles de chaîne A) sont additionnés une solution aqueuse de mercapto-2-éthanol de telle sorte que la concentration finale soit de 1 pour cent.

La solution est laissée au repos une heure puis dialysée en continu contre du tampon phosphate 125 mM pH 7 renouvelé pendant 40 heures à raison de 300 ml/heure. Selon la méthode d'Ellman, on a dosé 0,9 équivalent SH par mole de chaîne A de ricine.

On procède au blocage de cette fonction SH par le N-éthylmaléimide selon la méthode décrite dans Methods in Enzymology (11, 541, 1967). Pour cela, la chaîne A de ricine obtenue à l'étape précédente est incubée pendant 2 heures à 30°C en présence de 20 équivalents par mole de chaîne A de N-éthylmaléimide. On élimine l'excès de réactif par dialyse en continue contre du tampon phosphate 125 mM pH 7 renouvelé pendant 20 heures à raison de 500 ml/heure. On obtient ainsi 35 ml d'une solution de chaîne A de ricine à 7 mg/ml ne présentant plus de groupements thiol dosables par le réactif d'Ellman. Le produit ainsi obtenu est désigné ultérieurement sous l'appellation chaîne A (NEM).

b) Oxydation periodique de la chaîne A (NEM).
L'oxydation periodique de la chaîne A (NEM) est réalisée en utilisant la procédure indiquée dans l'exemple 2.

2) – Propriétés de la chaîne A (NEM) oxydée.
a) Activité enzymatique de la chaîne A (NEM) oxydée:
L'activité inhibitrice de la protéosynthèse a été déterminée en utilisant la procédure décrite dans l'exemple 1. On observe un maintien des propriétés enzymatiques avec une CI50 égale à $4,3.10^{-10}$ mole/l pour la chaîne A (NEM) oxydée.

b) Propriétés pharmacocinétiques de la chaîne A (NEM) oxydée.
La chaîne A (NEM) oxydée ou non est administrée chez le lapin par injection unique dans une veine de l'oreille. La quantité de chaîne A injectée correspond à 0,100 mg/kg. Les échantillons de plasma collectés au temps 23 h sont analysés à l'aide du test immunométrique IRM-1 tel que décrit dans l'exemple 2. Les résultats sont représentés dans le tableau suivant:

| | Concentration plasmatique à 23 h après l'injection |
|---|---|
| Chaîne A (NEM) | 0,01 % |
| Chaîne A (NEM) oxydée | 8 % |

Vingt trois heures après l'injection, la concentration de la chaîne A (NEM) oxydée est 800 fois supérieure à ce qu'elle est quand la chaîne A (NEM) n'est pas modifiée.

Exemple 4
Cet exemple démontre l'importance de la durée du traitement oxydatif sur les propriétés pharmacocinétiques de la chaîne A oxydée.
Six préparations de chaîne A oxydée sont réalisées en utilisant la procédure indiquée dans l'exemple 2 excepté pour la durée du traitement au periodate de sodium. Les temps de traitement sont les suivants: zéro; (arrêt immédiat par l'éthylène-glycol), 20 minutes, 40 minutes, 2,5 heures, 4 heures et 18 heures.

Ces différentes préparations sont injectées chez le lapin et la concentration plasmatique relative de la chaîne A est mesurée au temps 23 heures selon la même procédure que dans l'exemple 1.

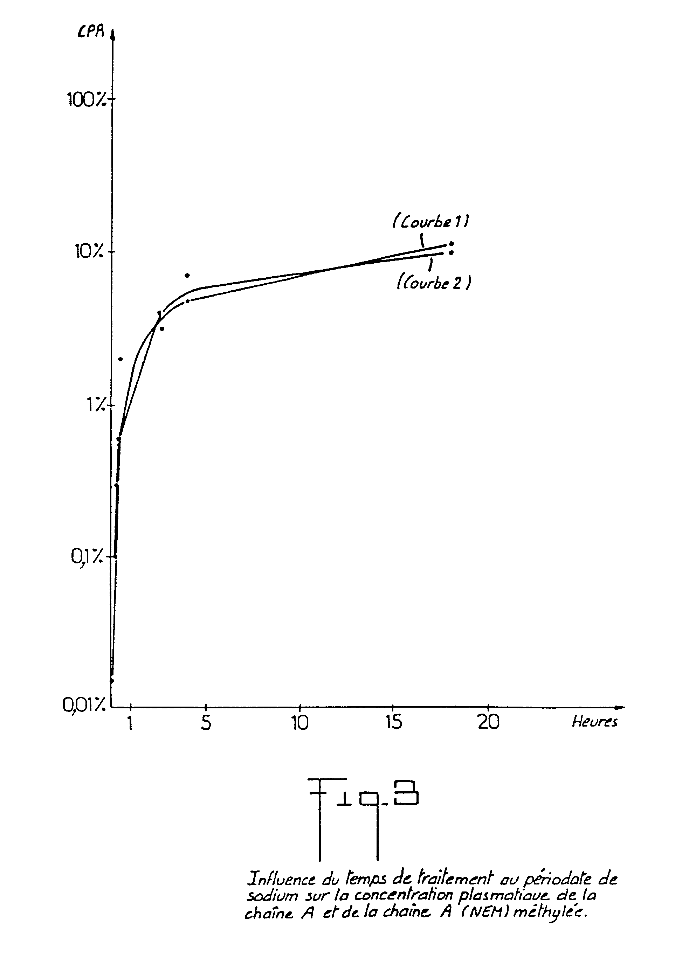

Les résultats sont représentés sur la figure 2, sur laquelle on a porté en abscisses la durée du traitement au périodate en heures et en ordonnées le CPR sur échelle logarithmique. Ces résultats indiquent que 1) l'accroissement du taux plasmatique de la chaîne A est bien dû à l'oxydation periodique puisque lorsque la réaction est immédiatement arrêtée la concentration plasmatique de chaîne A est identique à celle obtenue pour la chaîne A native, 2) il est nécessaire que la durée de cette réaction soit relativement longue pour obtenir des offets optimaux.

Exemple 5:

Cet exemple démontre l'importance de la durée du traitement oxydatif sur les propriétés pharmacocinétiques de la chaîne A bloquée au NEM et méthylée.

1) – Préparation de la chaîne A de ricine fonctionnalisée

a) Blocage du SH naturel de la chaîne A par le N-éthylmaléimide.

Le blocage du SH naturel de la chaîne A par le N-éthylmaléimide est réalisé selon la même procédure que celle décrite dans l'exemple 1.

b) Méthylation de la chaîne A.

La réaction de méthylation s'effectue à 0°C sous agitation dans du tampon borate 0,2M pH 10 selon la méthode de Means et Feeney (Biochemistry 7, 2192, 1968). A 65,5 ml de chaîne A (NEM) (3 mg/ml) on ajoute successivement 38 mg de borohydrure tritié (à 47 mCi/mole) puis 1 ml de formaldéhyde à 6% en 5 additions de 200 microlitres chacune, échelonnée sur un délai de 30 minutes.

On élimine l'excès de réactif par dialyse en discontinu contre du tampon phosphate 125 mM pH 7 (40 ml). Après dialyse la solution protéique est centrifugée. On recueille 63 ml de chaîne A méthylée à 3 mg/ml.

c) Oxydation periodique:

Six préparations de chaîne A NEM méthylée sont oxydées en utilisant la procédure indiquée dans l'exemple 1 excepté pour la durée du traitement au periodate de sodium. Les temps de traitement sont les suivants: zéro: (arrêt immédiat par l'éthylène-glycol), 10 minutes, 40 minutes, 2,5 heures, 4 heures et 18 heures.

Ces différentes préparations sont injectées chez le lapin et la concentration plasmatique relative de la chaîne A est mesuré au temps 23 heures selon la même procédure que dans l'exemple 2.

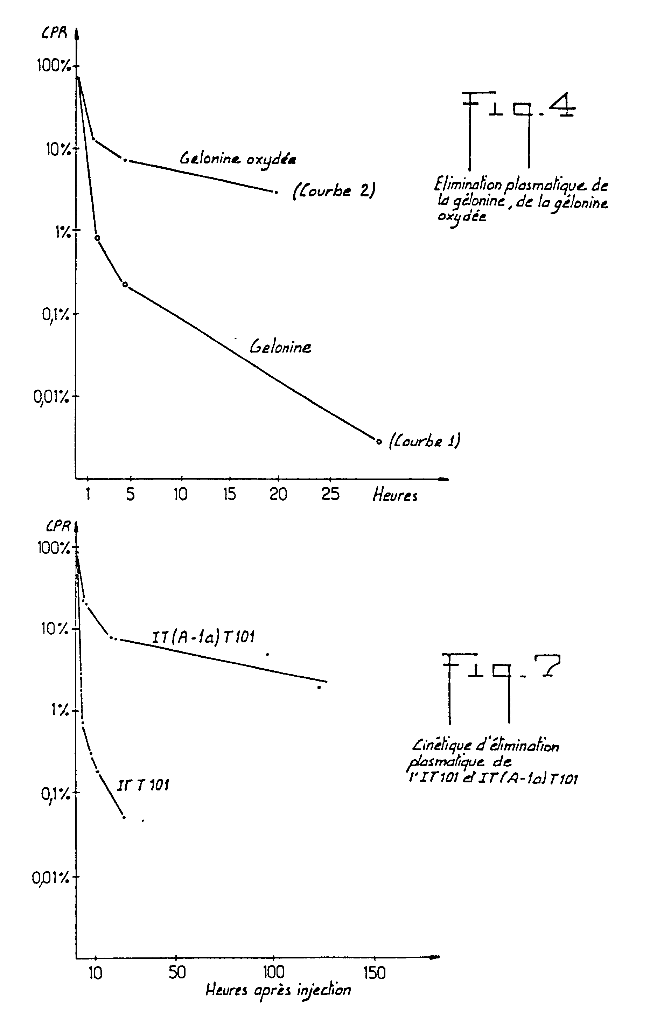

Les résultats sont représentés sur la figure 3, courbe 2. Ces résultats indiquent que comme pour la chaîne A (courbe 1):

1. l'accroissement du taux plasmatique de la chaîne A (NEM), méthylée, est bien dû à l'oxydation periodique puisque lorsque la réaction est immédiatement arrêtée la concentration plasmatique de chaîne A (NEM), méthylée, est identique à celle obtenue pour la chaîne A;

2. il est nécessaire que la durée de cette réaction soit relativement longue pour obtenir des effets optimaux.

Exemple 6:

Cet exemple démontre que la réaction d'oxydation réalisée séparément sur les deux variants moléculaires constitutifs de la chaîne A (chaîne A1 et chaîne A2) produit des effets, sur chacun des deux variants analogues à ceux décrits dans l'exemple 2 pour la chaîne A de ricine.

1) – Séparation des chaînes A1 et A2.

28 ml de chaîne A à 10,9 mg/ml (309 mg) dans le tampon phosphate 125 mM pH 7,0 sont déposés sur une colonne de 112 ml de concanavaline A-sépharose équilibrée dans le même tampon. La chaîne A1 est obtenue dans le premier pic par lavage avec le même tampon; la chaîne A2 est éluée par le tampon borate 0,1 M pH 6,0 + NaC1 0,5 M + alpha méthyl mannoside 0,1 M.

On obtient ainsi 184 mg de chaîne A1 et 103 mg de chaîne A2.

Les chaînes A1 et A2 sont concentrées par ultrafiltration sous pression d'azote; la chaîne A2 est dialysée contre le tampon phosphate 125 mM pH 7,0.

L'analyse de la chaîne A par électrophorèse en gel de gradient d'acrylamide avec SDS fait apparaître la présence de 2 bandes d'intensité différente correspondant à des poids moléculaires de 30000 et 33000. La chaîne A1 correspond à la bande de plus forte intensité de PM 30000, la chaîne A2 correspond à la bande de faible intensité de PM 33000.

2) – Modification des chaînes A1 et A2 de ricine par le periodate de sodium:

Cette modification est réalisée comme décrit dans l'exemple 2. Les préparations de chaîne A dont les motifs polysaccharidiques ont été oxydés, ont été étudiées en ce qui concerne leurs activités enzymatiques d'inhibition de la synthèse protéique et leurs propriétés pharmacocinétiques.

3) – Activités enzymatiques des chaînes A1 et A2 à longue durée d'action, mesurées sur un modèle acellulaire.

L'activité inhibitrice a été déterminée comme décrit dans l'exemple 1. On observe une CI50 égale à $2,1 \times 10^{-10}$ mole/l et à $2,1 \times 10^{-10}$ mole/l pour les chaînes A1 et A2 oxydées, respectivement. Les CI50 des chaînes A1 et A2 natives, témoins de l'expérience sont de $1,9 \times 10^{-10}$ mole/l et $1,10^{-10}$ mole/l, respectivement. La modification des variants séparés de la chaîne A n'entraîne donc pas de perte de leur activité enzymatique.

4) – Propriétés pharmacocinétiques des chaînes A1 et A2 à longue durée d'action (A1–La, A2–La).

La chaîne A1 ou A1–La ou A2 ou A2–La est administrée chez des lapins par injection unique dans une veine de l'oreille (415 microgrammes en chaîne A/kg). Les échantillons de plasma collectés au temps 20 heures sont analysés à l'aide du test immunométrique IRM-1 (voir exemple 2). Les résultats sont réprésentés dans le tableau suivant. Les valeurs de la chaîne A et A—La sont indiquées à titre de comparaison.

| | Concentration plasmatique relative à 20 heures après l'injection |
|---|---|
| Chaîne A | 0,012 % |
| Chaîne A oxydée (A—La) | 10 % |
| Chaîne A1 | 0,02 % |
| Chaîne A1 oxydée (A1—La) | 10 % |
| Chaîne A2 | 0,04 % |
| Chaîne A2 oxydée (A2—La) | 14 % |

Vingt heures après l'injection, la concentration en A1—La et A2—La est 500 et 350 fois supérieure à ce qu'elle est avec A1 et A2 respectivement.

Exemple 7:
Cet exemple décrit les caractéristiques biochimiques de la chaîne A et ses variants chaîne A1 et chaîne A2 sous forme native et sous forme oxydée.
Les chaînes A utilisées dans ces études sont préparées comme décrit dans les exemples 2 et 6.

I – Compositions en carbohydrates.
Les compositions en carbohydrates de ces protéines sont déterminées par analyses chromatographiques en phase gazeuse selon la méthode de Clamp (in Glycoproteins: their composition, structure and function (Gottschalk A., ed) vol 5 A, p 300—321, Elsevier Publishing Co., Amsterdam, London, New York).

Les résultats obtenus sont rassemblés dans les deux tableaux ci-dessus.

Composition centésimale

| Chaînes | Glucides totaux p. 100 |
|---|---|
| Natives A | 5,58 + ou — 0,5 |
| Natives A1 | 4,54 + ou — 0,5 |
| Natives A2 | 6,24 + ou — 0,5 |
| Oxydées A | 2,27 + ou — 0,5 |
| Oxydées A1 | 2,07 + ou — 0,5 |
| Oxydées A2 | 3,33 + ou — 0,5 |

Composition molaire
(sur la base d'une masse moléculaire de 30625 les résultats sont donnés avec une précision moyenne de + ou — 0,5 résidus par molécule).

| Monosaccharides | Chaînes Natives A | A1 | A2 | Oxydées A | A1 | A2 |
|---|---|---|---|---|---|---|
| N-acétyl-glucosamine | 1,89 | 1,48 | 2,15 | 1,74 | 1,50 | 2,37 |
| Mannose | 4,6 | 3,40 | 5,2 | 1,43 | 1,29 | 2,26 |
| Fucose | 1,37 | 1,41 | 1,52 | 0 | 0 | 0 |
| Xylose | 1,6 | 1,48 | 1,67 | 0,36 | 0,48 | 0,62 |

Ces résultats prouvent que l'oxydation periodique a détruit une partie des sucres de la chaîne A. Par molécule de chaîne A, on note une diminution moyenne de 3,17, 2,11 et 2,94 résidus mannose, la disparition complète des résidus fucose et une diminution moyenne de 1,24, 1 et 1,05 résidus de xylose pour les chaînes A, A1 et A2 respectivement. Les résidus de N-acétylglucosamine, quant à eux, ne sont que peu altérés.

II – Séquence N-terminale.
La séquence des acides aminés N-terminaux de la chaîne A et de ses variants A1 et A2 sous forme native et sous forme oxydée a été établie avec une séquenceur de protéines selon les procédures connues par l'homme de l'art. Les résultats obtenus sont rassemblés dans le tableau suivant.

| Chaînes | Séquence des 9 acides aminés N-terminaux |
|---|---|
| Natives A | Ile-Phe-Pro-Lys-Gln-Tyr-Pro-Ile-Ile |
| Natives A1 | Ile-Phe-Pro-Lys-Gln-Tyr-Pro-Ile-Ile |
| Natives A2 | Ile-Phe-Pro-Lys-Gln-Tyr-Pro-Ile-Ile |
| Oxydées A | Ile-Phe-Pro-Lys-Gln-Tyr-Pro-Ile-Ile |
| Oxydées A1 | Ile-Phe-Pro-Lys-Gln-Tyr-Pro-Ile-Ile |
| Oxydées A2 | Ile-Phe-Pro-Lys-Gln-Tyr-Pro-Ile-Ile |

On constate que les séquences des 9 acides aminés N-terminaux des chaînes A, A1 et A2 natives et oxydées sont rigoureusement identiques entre elles, ce qui démontre que le traitement oxydatif laisse intacte la chaîne protéique. On constate également que la séquence des 9 acides aminés N-terminaux des chaînes A est rigoureusement identique à celle décrite antérieurement par Funatsu pour la chaîne A de ricine (Agric. Biol. Chem., (1979), 43, 2221).

III – Affinité sur concanavaline A sépharose:

Les chaînes A, A bloquée au DTNB [A(DTNB)], A(DTNB)-La, A1(DTNB), A1(DTNB7-La, A2(DTNB), A2(DTNB)-La sont testées par leur capacité à se fixer sur la concanavaline A sepharose. 1 ml de solution de chaîne A, à environ 1 mg/ml est déposée sur une colonne de 1 ml de concanavaline A. La chromatographie est suivie par la lecture de la densité optique à 280 nm. Après lavage par le tampon phosphate 125 mM pH 7,0 jusqu'au retour à la ligne de base du premier pic non retenu par la concanavaline A, la colonne est lavée par le tampon borate 0,1 M pH 6,0 contenant du NaCl 0,5 M

| Chaînes | % non retenu par la con A | % élué par alpha méthylmannoside | Total (%) |
|---|---|---|---|
| A | 53 | 27 | 80 |
| A(DTNB) | 66 | 11 | 77 |
| A(DTNE)-La | 78 | 5 | 83 |
| A1(DTNB) | 80 | 6 | 86 |
| A1(DTNB)-La | 73 | 0,4 | 73,4 |
| A2(DTNB) | 25 | 70 | 95 |
| A2(DTNB)-La | 64 | 9 | 73 |

Il est connu que la concanavaline A présente de l'affinité pour les glycoprotéines à mannoses terminaux. On constate que la chaîne A qui contient de tels résidus peut se lier à la con A. Ceci est particulièrement net dans le cas de la chaîne A2 qui est l'isomère qui est le plus richement substitué en mannose. On constate aussi que le traitement oxydatif dissipe cette affinité, ce qui est cohérent avec la destruction des résidus sucrés par un tel traitement.

IV – Détermination du E 1‰.

Le coefficient d'absorption à 280 nm (E 1‰) est la densité optique à 280 nm d'une solution à 1 mg/ml dont la concentration en protéine est déterminée par le dosage FOLIN avec une gamme étalon de sérum albumine bovine.

Les résultats sont résumés dans le tableau suivant:

| Chaîne A | 0,65 |
|---|---|
| Chaîne A(DTNB) | 1,12 |
| Chaîne A(DTNB)L–a | 1,04 |
| Chaîne A1(DTNB) | 1,07 |
| Chaîne A1(DTNB)L–a | 1,02 |
| Chaîne A2(DTNB) | 0,95 |
| Chaîne A2(DTNB)L–a | 0,95 |

Le blocage de la fonction thiol de la chaîne par le DTNB fait apparaître une augmentation sensible de l'absorption à 280 nm due à l'introduction du groupement nitrobenzoyl.

Après oxydation, on n'observe pas de variation significative de l'absorption à 280 nm ce qui démontre que l'oxydation n'a pas touché d'acides aminés responsables de l'absorption à 280 nm.

V – Isoélectrofocalisation.

L'analyse de la chaîne A par isoélectrofocalisation fait apparaître un ensemble de bandes de points isoélectriques (pI) compris entre 7,5 et 8,0 et identiques pour les chaînes A, A1 et A2.

Le blocage de la cystéine de la chaîne de la chaîne A par le DTNB provoque un élargissement des bandes vers la zone acide; la libération de la cystéine par le mercaptoéthanol ramène ces bandes au niveau de la chaîne A native.

et de l'alpha méthyl mannoside 0,1 M. Les résultats exprimés en pourcentage de la densité optique à 280 nm sont résumés dans le tableau suivant.

Ainsi, la comparaison des points isoélectriques des chaînes A, A1, A2 natives et oxydées fait apparaître, en l'absence d'agent bloquant, un transfert de 0,5 unité de pH vers les pH acides de l'ensemble des bandes caractéristiques de la chaîne A. Ce transfert se fait sans recouvrement des zones de pH des chaînes A natives et oxydées, ce qui semble indiquer que toutes les molécules de chaîne A sont touchées par l'oxydation.

Exemple 8:

Cet exemple démontre, après injection intraveineuse chez l'animal, 1) l'élimination rapide de la gélonine native et 2) l'élimination lente de la gélonine modifiée par le periodate de sodium.

I – Modification de la gélonine par le periodate de sodium.

La gélonine a été préparée et purifiée à partir de Gelonium multiflorum selon la méthode décrite (J. Biol. Chem. (1980) 255, 6947–6953). La réaction d'oxydation est réalisée dans les mêmes conditions que celles décrites pour la chaîne A de ricine dans l'exemple 2 excepté que l'étape de blocage des thiols par le DTNB n'est pas pratiquée.

En effet, le couplage de la gélonine à l'anticorps n'étant pas généralement pratiqué à partir de groupements thiol naturels de la gélonine, les groupements thiols seront introduits artificiellement après l'étape d'oxydation selon la technique décrite dans Cancer Res., 1984, 44, 129–133. A 1 ml de solution à 3 mg/ml de gélonine dans le tampon PBS, amenée à pH 6 à l'aide d'acide acétique 1 M, on ajoute 21 microlitres d'une solution de periodate de sodium 0,5 M dans l'eau. On laisse l'incubation se poursuivre pendant 16 heures à 4°C dans l'obscurité. La réaction est arrêtée par addition de 105 microlitres d'une solution aqueuse d'éthylène-glycol 1 M. Après 15 minutes d'incubation à 20°C, le milieu réactionnel est dialysé à 4°C contre du tampon PBS. On obtient ainsi après centrifugation à 10 000 × g pendant 30 minutes 2,9 mg de gélonine oxydée à une concentration de 2,5 mg/ml.

Comme la chaîne A de ricine, la propriété fondamentale de la gélonine est d'inhiber la synthèse protéique de cellules eucaryotes par altération de la sous-unité ribosomale 60 S (Biochem. J. (1982) 207, 505–509). Dans le cas de la gélonine aussi, la modification due à l'oxydation periodique n'entraîne pas de perte d'activité.

II – Propriétés pharmacocinétiques de la gélonine à longue durée d'action.

La gélonine native ou modifiée selon les procédures développées ci-dessus est administrée chez le lapin par injection unique dans une veine de l'oreille. La quantité de gélonine injectée est comprise entre 0,3 et 0,4 mg/kg. Les échantillons de sang sont prélevés au cours du temps sur héparine. Les plasmas sont analysés à l'aide d'un test immunoradiométrique ci-après désigné par l'abréviation IRM 2.

Cet essai est réalisé selon la même technique que pour le test IRM 1 excepté que la solution Ac1 est ici une solution d'anticorps de lapin anti-gélonine pruifiés par chromatographie d'affinité, les anticorps Ac2 étant les mêmes anticorps radiomarqués. La procédure de radiomarquage est identique à celle décrite pour la technique IRM 1. La mesure de la concentration en gélonine native ou gélonine modifiée dans les échantillons à doser se fait par référence à une courbe d'étalonnage réalisée avec la gélonine native ou modifiée introduite à différentes concentrations connues. L'essai IRM-2 répond aux mêmes caractéristiques de fiabilité et reproductibilité que celles décrites pour la technique IRM-1. Les résultats de ces expériences sont représentés de la même façon que pour la chaîne A de ricine dans l'exemple 2.

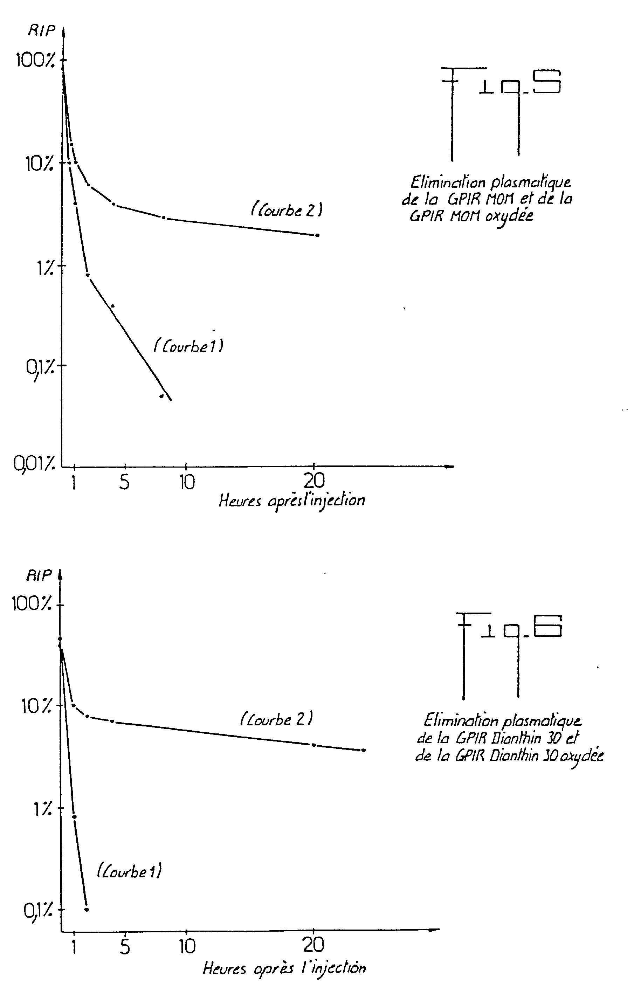

La figure 4 montre les courbes d'élimination plasmatique, en fonction du temps, de la gélonine native et de la gélonine oxydée injectées par voie intraveineuse. La gélonine native, comme la chaîne A de ricine native, disparaît très rapidement de la circulation puisque 99,99% de la gélonine présente dans le sang circulant disparaissent en 24 heures (courbe 1). Lorsque la gélonine a été oxydée sur ses motifs polysaccharidiques, le profil d'élimination est profondément modifié: 24 heures après l'injection la concentration de la gélonine oxydée est 300 fois supérieure à celle de la gélonine native (courbe 2).

Ainsi, comme pour la chaîne A de ricine, ces résultats prouvent que l'oxydation periodique a modifié les sucres impliqués dans le processus de reconnaissance responsable de l'élimination de la gélonine au point d'empêcher cette reconnaissance.

Exemple 9:

Cet exemple démontre, après injection intraveineuse chez l'animal:

1. l'élimination rapide de la GPIR MOM extraite de Momordica charantia et

2. l'élimination lente de la GPIR MOM modifiée par le periodate de sodium.

1) – Modification de la GPIR MOM par le periodate de sodium.

La GPIR MOM a été préparée et purifiée à partir de l'endosperme des graines Momordica charantia selon la méthode décrite (Biochemical Journal (1980), 186, 443–452). Les propriétés pharmacocinétiques du MOM natif ou modifié ont été établies en utilisant le GPIR MOM radioactif. Le MOM est radiomarqué sur les tyrosines avec de l'iode radioactif 125 en présence de chloramine T. A 100 microlitres de solution à 1 mg/ml de MOM dans le tampon PBS, on ajoute 10 microlitres d'une solution d'iode radioactif 125 à 100 μ Ci/ml et 30 microlitres d'une solution de chloramine T à 2,5 mg/ml dans l'eau. On laisse la réaction se poursuivre pendant une minute à température ambiante. La réaction est arrêtée par addition de 400 microlitres d'une solution de sodium métabisulfite à 0,5 mg/ml. Le milieu réactionnel est chromatographié par gel filtration sur une colonne de Sephadex G25 avec du tampon PBS contenant 0,1% de gélatine pour séparer la protéine radiomarquée de l'iode qui n'a pas réagi. On obtient ainsi, après centrifugation à 10000 xg pendant 30 minutes, 80 microgrammes de MOM radiomarquée à une concentration de 0,04 mg/ml.

La réaction d'oxydation est réalisée dans les mêmes conditions que celles décrites pour la chaîne A de ricine dans l'exemple 2 excepté que l'étape de blocage des thiols par le DTNB n'est pas pratiquée et que la concentration en protéine est de 125 fois (40μg/ml) plus faible. A 1 ml de solution à 0,04 mg/ml de MOM radiomarquée, amenée à pH 6 à l'aide d'acide acétique 1 M, on ajoute 20 microlitres d'une solution de periodate de sodium 0,5 M dans l'eau. On laisse l'incubation se poursuivre pendant 16h à 4°C dans l'obscurité. La réaction est arrêtée par addition de 100 microlitres d'une solution aqueuse d'éthylène-glycol 1 M. Après 15 minutes d'incubation à 20°C le milieu réactionnel est dialysé à 4°C contre du tampon PBS. On obtient après centrifugation à 10000 xg pendant 30 minutes, 32 microgrammes de MOM oxydée à une concentration de 0,021 mg/ml.

La nouvelle molécule ainsi obtenue possède un poids moléculaire non significativement différent de celui de la MOM native. Le procédé de modification ne fait apparaître de façon visible, par électrophorèse en gradient de polyacrylamide après révélation par le bleu de coomassie ou autoradiographie, des polymères de la protéine qu'en très faible quantité et aucun produit de dégradation.

2) – Propriétés pharmacocinétiques du MOM à longue durée d'action:

Le MOM radiomarqué oxydé ou non selon les procédures développées ci-dessus est administré chez le lapin par injection unique dans une veine de l'oreille. La quantité de MOM injectée est comprise entre 3,50 et 3,55 microgrammes/kg. Les échantillons de sang sont prélevés au cours du temps sur héparine. Les plasmas (200 microlitres) sont incubés avec de l'acide trichloracétique (TCA) (200 microlitres à 25%) pendant 30 minutes à 4°C. Après centrifugation la radioactivité acide précipitable contenue dans le culot est déterminée. Cette méthode d'analyse permet de mesurer le taux plasmatique des molécules de MOM intactes, les produits éventuels de dégradation, de petits poids moléculaire, qui ne sont pas précipitables par le TCA, ne sont pas pris en compte.

Les résultats de ces expériences sont représentés par le pourcentage de la radioactivité initiale demeurant dans la circulation en fonction du temps. Cette valeur appelée «pourcentage de la radioactivité initiale plasmatique» (% RIP) est calculée à l'aide de l'expression suivante:

$$\% \text{ R.I.P.} = \frac{r \times VP \times 100}{0,2 \times R}$$

où:

r = radioactivité mesurée au temps t dans 0,2 ml de plasma,
R = radioactivité totale injectée,
VP = volume plasmatique (considéré égal à 36 ml/kg de poids corporel de l'animal).

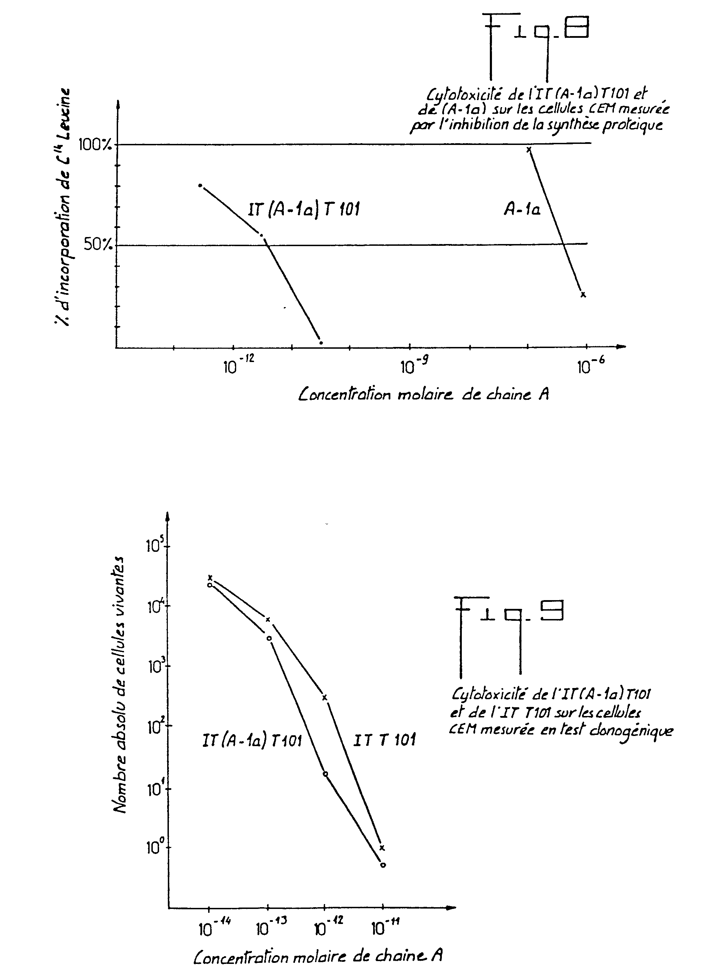

Les courbes d'élimination plasmatiques, en fonction du temps, du MOM oxydé ou non après injection intraveineuse sont représentées dans la figure 5. Le MOM, comme la chaîne A de ricine native, disparaît très rapidement de la circulation puisque 99,9% du MOM présent dans le sang circulant disparaissent en 8 heures (courbe 1). Lorsque le MOM a été oxydé sur ses résidus sucrés, la vitesse d'élimination est ralentie (courbe 2): 8 heures après l'injection le taux de MOM oxydé est 60 fois supérieur à celui du MCM non oxydé. Ces résultats prouvent que l'oxydation periodique a modifié les sucres impliqués dans le processus de reconnaissance responsable de l'élimination rapide du MOM.

Exemple 10:
Cet exemple démontre, après injection intraveineuse chez l'animal:
1. l'élimination rapide de la GPIR Dianthin extraite de Dianthus caryophyllus et
2. l'élimination lente de la GPIR Dianthin modifiée par le periodate de sodium.

1) – Modification de la Dianthin 30 pajr le periodate de sodium.
La Dianthin 30 a été préparée et purifiée à partir des feuilles de Dianthus caryophyllus selon la méthode décrite [Biochemical Journal (1981), 195, 339–405). Les propriétés pharmacocinétiques de la Dianthin 30 oxydée ou non ont été établies en utilisant la Dianthin radioactive. Les réactions d'iodination et d'oxydation sont réalisées dans les mêmes conditions que celles décrites pour le MOM dans l'exemple 9.
La nouvelle molécule de Dianthin oxydée ainsi obtenue possède un poids moléculaire non significativement différent de celui de la Dianthin 30 native.

2) – Propriétés pharmacocinétiques de la Dianthin 30 à longue durée d'action.
La Dianthin radiomarquée oxydée ou non est administrée chez le lapin par injection unique dans une veine de l'oreille. La mesure du taux plasmatique de la Dianthin est réalisée selon le même mode opératoire que celui décrit pour le MOM dans l'exemple 9. La figure 6 représente les courbes d'élimination plasmatiques, en fonction du temps, de la Dianthin oxydée (courbe 2) ou non (courbe 1). La Dianthin 30, comme le MOM, disparaît très rapidement de la circulation sanguine puisque 99,9% de la quantité présente initialement disparaissent en 2 heures. Par contre lorsque la Dianthin 30 a été oxydée sur les résidus carbohydrates, la cinétique d'élimination est fortement ralentie: 2 heures après l'injection le taux de Dianthin est 80 fois supérieur à celui de la Dianthin non oxydée. Le taux de Dianthin oxydée reste élevé au delà de 24 heures (3% de la valeur initiale à 24 heures).

Ici encore ces résultats prouvent que l'oxydation periodique a modifié les sucres impliqués dans le processus de reconnaissance responsable de l'élimination rapide de la Dianthin.

Exemple 11:
Conjugué obtenu par réaction entre un anticorps anti-cellules T humaines (anticorps dirigé contre l'antigène T65) substitué par des groupements disulfure activé et la chaîne A de ricine oxydée.
a) Anticorps anti-cellules T humaines (ou anticorps T101).
Cet anticorps été obtenu selon la méthode décrite dans Journal of Immunology, 1980, 125 (2), 725–737.
b) Chaîne A de ricine oxydée: La chaîne A de ricine a été préparée ainsi qu'il a été indiqué dans l'exemple 2.

II) Anticorps activés anti-cellules T humaines
A 100 microlitres d'une solution à 20 mg/ml d'acide (pyridyl 2 disulfanyl)-3 propionique dans le tertiobutanol, on ajoute 20 microlitres d'une solution à 60,3 mg/ml d'éthyl-1 (dimethylamino-3 propyl)-3 carbodiimide et on laisse 3 minutes à température ambiante. On ajoute 68 microlitres de la solution ainsi obtenue à 2 ml d'une solution d'anticorps à 8,9 mg/ml dans le tampon PBS. Le mélange est agité 15 minutes à 30°C puis dialysé contre du tampon PBS à 4°C. Après dialyse la solution protéique est centrifugée et l'on obtient ainsi 15 mg d'anticorps activé à une concentration de 7,9 mg/ml. Par dosage spectrophotométrique à 343 nm de la pyridine thione-2 libérée par échange avec le mercapto-2 éthanol, on constate que l'on a obtenu un anticorps portant 3,8 groupements disulfure mixte activé par mole d'anticorps.

III) Préparation de l'immunotoxine à chaîne A de ricine à longue durée d'action
A 1,5 ml de la solution d'anticorps activé précédemment obtenue (concentration 7,9 mg/ml, soit 11,8 mg d'anticorps activés), on ajoute 2,46 ml de chaîne A modifiée à 2,87 mg/ml et on incube 20 heures à 20°C. La solution est centrifugée puis purifiée par filtration sur colonne de Séphadex G 100 avec mesure de la densité optique de l'effluent à 280 nm. Le regroupement des fractions contenant à la fois l'anticorps et la chaîne A conduit à 15 ml de solution d'immunotoxine à 0,7 mg/ml soit 10,5 mg. Cette solution contient 0,14 mg de chaîne A oxydée couplée à l'anticorps par ml.

Le taux de couplage moyen de cette préparation est donc de 1,2 mole de chaîne A oxydée par mole d'anticorps.

L'immunotoxine à chaîne A de ricine oxydée, IT (A−1a) T101 obtenue comme indiqué ci-dessus a été étudiée en ce qui concerne ses propriétés pharmacocinétiques et ses propriétés de cytotoxicité spécifique vis-à-vis des cellules cibles.

Exemple 12:

Cet exemple démontre l'acquisition de la propriété de lente élimination plasmatique des immunotoxines à chaîne A de ricine à longue durée d'action désignées en abrégé IT (A−1a) T101.

I – Mode opératoire

Le conjugué préparé selon la procédure développée dans l'exemple 11 est administré chez le lapin par injection unique dans une veine de l'oreille. La quantité injectée correspond à 0,415 mg/kg exprimé en chaîne A. Les échantillons de sang sont prélevés au cours du temps sur héparine. Les plasmas sont analysés à l'aide d'un test immunoradiométrique à deux sites ci-après désigné par l'abréviation IRM-3.

Cet essai est réalisé selon la même technique que pour le test IRM-1 excepté que la solution Ac2 est ici une solution d'anticorps de chèvres anti-IgG de souris purifiés par chromatographie d'affinité et radiomarqués comme décrit pour la technique IRM-1. La mesure de la concentration en immunotoxine modifiée dans les échantillons à doser se fait par référence à une courbe d'étalonnage réalisée avec l'immunotoxine modifiée introduite à différentes concentrations connues. L'essai IRM-3 répond aux mêmes caractéristiques de fiabilité et de reproductibilité que celles décrites pour la technique IRM-1.

Pour comparaison une étude contrôle est réalisée dans les mêmes conditions avec le conjugué appelé IT-T101 obtenu par réaction entre le même anticorps T101 substitué par des groupements disulfure activé et la chaîne A native de ricine. La préparation et les propriétés cytotoxiques de ce conjugué ont été décrites dans la demande de brevet français n° 81/21 836. Les résultats de ces expériences sont représentés de la même façon que pour la chaîne A de ricine non couplée dans l'exemple 2.

II – Résultats

La figure 7 montre les courbes d'élimination plasmatique en fonction du temps de 1'IT T101 et de 1'IT (A−1a) T101 injectées par voie intraveineuse. Vingt quatre heures après l'injection, la concentration d'immunotoxine active est 140 fois supérieure pour 1'IT (A−1a) T101 que pour 1'IT T101. Ce fait démontre que les nouvelles propriétés pharmacocinétiques de la chaîne A oxydée sont conservées après couplage à un anticorps.

Exemple 13:

Cet exemple démontre le maintien des propriétés de cytotoxicité spécifique de 1'IT (A−1a) T101 vis-à-vis des cellules-cibles.

La propriété biologique fondamentale de la chaîne A de ricine est d'inhiber la synthèse protéique cellulaire par altération de la sous-unité ribosomale 60S. La technique utilisée met en œuvre un modèle cellulaire dans lequel on mesure l'effet des substances étudiées sur l'incorporation de 14C-leucine dans les cellules cancéreuses en culture.

Les cellules utilisées appartiennent à la lignée cellulaire CEM issue d'une leucémie T humaine qui porte l'antigène T65. Les cellules sont incubées en présence de la substance à étudier puis, en fin d'incubation, on procède à la mesure du taux d'incorporation de 14C-leucine par les cellules ainsi traitées.

Cette mesure est effectuée selon une technique adaptée de la technique décrite dans Journal of Biological Chemistry 1974, 249 (11), 3557−3562 utilisant le traceur 14C-leucine pour la détermination du taux de synthèse protéique. La détermination de la radioactivité incorporée est effectuée ici sur les cellules entières isolées par filtration.

A partir de ces déterminations, on peut tracer les courbes effets/doses présentant en abscisses la concentration molaire en chaîne A des substances étudiées et en ordonnées l'incorporation de 14C-leucine exprimée en pourcentage de l'incorporation des cellules témoins en l'absence de toute substance affectant la synthèse protéique.

On peut ainsi déterminer pour chaque substance étudiée la concentration qui inhibe 50% de l'incorporation de 14C-leucine ou «concentration inhibitrice 50» (CI 50).

La figure 8 représente les courbes obtenues dans la même expérience avec 1'IT-(A−1a)-T101 et la chaîne A oxydée non couplée en présence de chlorure d'ammonium 10 mM dans le milieu d'incubation. On peut constater sur cette figure que 1'IT-(A−1a)-T101 a une très forte activité cytotoxique (CI50 = 5,5 × $10^{-12}$M) environ 80000 fois supérieure à celle de la chaîne A oxydée non couplée mesurée dans les mêmes conditions.

Exemple 14:

Cet exemple démontre l'efficacité cytotoxique comparée de 1'IT (A−1a) T101 et de 1'IT T101 vis-à-vis des cellules cibles CEM, mesurée dans un test clonogénique.

La vocation des immunotoxines est l'éradication des cellules cibles jusqu'à la dernière. Cette performance ne peut être évaluée qu'avec une technique d'une grande sensibilité: les tests d'inhibition de formation de colonies offrent cette possibilité puisqu'une seule cellule survivante peut être visualisée parmi plusieurs millions de cellules mortes. Ceci est rendu possible par des conditions de culture optimales en milieu gélifié, appliquées à la lignée lymphoïde humaine CEM.

I – Technique de mesure de la cytotoxicité par inhibition de la formation de colonies.

Le milieu utilisé pour le clonage est le milieu RPMI 1640 auquel on ajoute 1 mM d'alpha-céto-glutarate de sodium, 1 mM d'oxaloacétate de sodium, 5% de sérum de veau fœtal inactivé et 10% de sérum de cheval inactivé. Une première solution d'agar à 0,3% (Agarose type VII, laboratoires SIGMA) est préparée dans ce milieu, disposée en couche mince dans de petites boîtes de Pétri et solidifiée a + 4°C. Les cellules sont mélangées à une seconde solution d'agar à 0,275% maintenue à 37°C, déposée ensuite sur la première couche et solidifiée. Ces concentrations d'agar ont été chosies après une étude préliminaire visant à optimiser à la fois l'efficacité de clonage, la dimension des colonies et la consistance du milieu. Après 15 jours en incubateur, les colonies sont dénombrées à l'aide d'un compteur automatique de colonies («ARTEK», DYNATECH, U.S.A.). L'établissement d'une droite d'étalonnage où sont portés le nombre de cellules ensemencées en fonction du nombre de colonies formées est indispensable pour déterminer l'efficacité du clonage et ainsi le nombre exact de cellules survivant au traitement par l'immunotoxine. Nous avons vérifié que l'efficacité du clonage réflétée par cette droite étalon n'est pratiquement pas influencée par la présence d'une proportion élevée de cellules mortes, situation naturellement rencontrée quand les cellules sont traitées par l'immunotoxine.

Le traitement à l'immunotoxine est réalisé par incubation des cellules en phase de croissance exponentielle et à la concentration de $10^6$/ml avec l'immunotoxine IT (A−1a) T101 ou ITT101 à différentes concentrations, sous un volume total de 1 ml de milieu RPMI-1640 contenant 10% de sérum de veau fœtal inactivé, et 10 mM de chlorure d'ammonium. L'incubation a lieu à 37°C, sous atmosphère contenant 5% de gaz carbonique et avec agitation horizontale des tubes tests (2 500 tours par minute avec agitateur «GIRATORY G-2», NEW-BRUNSWICK). Les cellules sont ensuite lavées et différentes dilutions sont réalisées avant mélange à la solution d'agar, de façon que la lecture du nombre de cellules survivantes puisse être faite dans la zone de sensibilité maximale donnée par la droite étalon. Les résultats sont exprimés par le nombre absolu de cellules surivantes extrapolé à partir de l'efficacité de clonage, en utilisant la relation suivante:

$$\text{nombre absolu de cellules survivantes: } \frac{C \times d}{E}$$

où C est le nombre de clones par boîte de Pétri, d est le facteur de dilution de la préparation cellulaire examinée, et E est l'efficacité de clonage établie à partir de la pente de la droite d'étalonnage. Chaque point correspond à la moyenne de trois essais.

II – Résultats

La figure 9 montre les courbes d'activité cytotoxique sur les cellules CEM des immunotoxines IT (A−1a) T101 et IT T101 en présence de chlorure d'ammonium à 10 mM en fonction de la concentration en immunotoxine (exprimée en molarité de chaîne A). Il apparaît que les efficacités de ces deux produits sont du même ordre de grandeur. La cytoréduction obtenue est extrêmement importante dans les deux cas puisque pour des concentrations aussi faibles que $10^{-11}$ M le taux de cellules survivantes résiduelles est de l'ordre de 0,001% de la valeur initiale. Cet effet est hautement spécifique puisque, à ces concentrations, il a été vérifié que la chaîne A non couplée ou une immunotoxine non spécifique sont sans effet sur ces cellules.

Cet exemple démontre que 1'IT (A−1a) T101 a des propriétés de cytotoxicité spécifique pratiquement identiques à celles de 1'IT T101 conventionnelle.

Exemple 15:

Conjugué obtenu par réaction entre un anticorps anti-cellules T humaines (anticorps dirigé contre l'antigène T 65) substitué par des groupements disulfure activés et la chaîne A de ricine (NEM), oxydée et fonctionnalisée; le couplage s'opérant entre les groupements disulfure activés et les résidus sucrés fonctionnalisés de la chaîne A.

1) – Préparation de l'immunotoxine.

a) Préparation de la chaîne A fonctionnalisée.

La chaîne A est bloquée par le N-éthylmaléimide sur son groupement SH puis oxydée pendant 18 heures selon la procédure décrite dans l'exemple 3.

Couplage à la cystamine:

Après dialyse contre du tampon carbonate pH 9,5, 5,2 ml de la solution protéique à 4,65 mg/ml sont mis à incuber avec 18 mg de chlorhydrate de cystamine pendant 2 heures à 25°C. Cette incubation est suivie d'une réduction au borohydrure de sodium (200 équivalents par mole de chaîne A), soit 156 microlitres d'une solution à 17,6 mg dans 1 ml de NaOH 0,1 N pendant 2 heures à 25°C.

On élimine l'excès de réactif par dialyse en continu contre du tampon phosphate 125 mM pH 7. Le pont disulfure de la cystamine fixée est ensuite réduit par le mercapto-2 éthanol à la concentration finale de 5 pourcent pendant 1 heure à 30°C. On poursuit ensuite la dialyse en continu contre du tampon phosphate 125 mM pH 7 (20 l à 300 ml/heure). Après dialyse et centrifugation on a dosé selon la méthode Ellman, 0,25 SH par mole de chaîne A.

b) Préparation de l'anticorps (voir exemple 11).

c) Réaction de couplage.

A 211 microlitres de la solution d'anticorps activé précédemment obtenue (soit 0,006 micromole) on ajoute 1,5 ml de la solution de chaîne A de ricine modifiée (soit 0,058 micromole). On laisse le mélange évoluer 18 heures à 30°C. Le milieu réactionnel est ensuite dialysé contre du tampon PBS (phosphate 10 mM, chlorure de sodium 140 mM pH 7,4). Après centrifugation et examen par électrophorèse en gradient de polyacrylamide, on constate que l'on a obtenu une immunotoxine dont le taux de couplage moyen est pour cette préparation de 0,8 chaîne A (NEM) par mole d'anticorps.

2) – Propriétés de l'immunotoxine IT [A (NEM)-La-cysteamine] T 101.

Activité de cytoxicité spécifique:
On constate que cette immunotoxine préparée selon la procédure développée ci-dessus a une très forte activité cytotoxique sur les cellules cibles CEM (CI 50 = 1,2 × 10$^{-11}$M; établie selon la méthode décrite dans l'exemple 13).

b) Elimination plasmatique:
L'immunotoxine est administrée chez le lapin par injection unique dans une veine de l'oreille (50 microgrammes en chaîne A/kg). Les échantillons de plasma collectés au temps 22 heures sont analysés à l'aide du test immunométrique IRM-3 (exemple 12). Les résultats sont représentés dans le tableau suivant. Les valeurs de 1'IT T101 sont indiquées à titre de comparaison.

| | Concentration plasmatique ralative à 22 heures après l'injection |
|---|---|
| IT [A(NEM)-La-cystéamine) T 101 | 2,4 % |
| IT T 101 | 0,08% |

Vingt deux heures après l'injection, la concentration en IT à chaîne A modifiée est 30 fois supérieure à ce qu'elle est avec 1'IT T101.

Exemple 16:
Conjugué obtenu par réaction entre un anticorps anti-cellules T humaines (anticorps dirigé contre l'antigène T 65) substitué par des groupements disulfure activés et la chaîne A de ricine NEM, méthylée, oxydée et fonctionnalisée, le couplage s'opérant entre les groupements disulfure activés et les résidus sucrés modifiés de la chaîne A.

1) – Préparation de l'immunotoxine.
a) Préparation de la chaîne A fonctionnalisée.
La chaîne A est bloquée par le N-éthylmaléimide sur son groupement SH puis méthylée et oxydée pendant 18 heures selon la méthode décrite dans l'exemple 5.

Couplage à la cystamine:
Après dialyse contre du tampon carbonate 0,1 M pH 9,5, 18,5 ml de la solution protéique à 2,5 mg/ml sont mis à incuber avec 35,6 mg de chlorhydrate de cystamine pendant 2 heures à 25°C. Cette incubation est suivie d'une réduction au borohydrure de sodium (200 équivalents par mole de chaîne A), soit 395 microlitres d'une solution à 17,6 mg dans 1 ml de NaOH 0,1 N, pendant 2 heures à 25°C.

On élimine l'excès de réactif par dialyse en continu contre du tampon phosphate 125 mM pH 7. Le pont disulfure de la cystamine fixée est ensuite réduit par le mercapto-2-éthanol à la concentration finale de 5 pourcent pendant 1 heure à 30°C. On poursuit ensuite la dialyse en continu contre du tampon phosphate 125 mM pH 7 (201 à 300 ml/heure). Après dialyse et centrifugation on a dosé selon la méthode de Ellman 0,32 SH par mole de chaîne A.

b) Préparation de l'anticorps modifié.
A 23,5 ml d'une solution d'anticorps T 101 à 4,4 mg/ml (soit 0,68 micromoles) est ajouté une solution contenant 2,12 mg de N-succinimidyl-3(2-pyridyldithio) propionate dans l'alcool éthylique. Le mélange est agité 30 min à 25°C puis dialysé contre du tampon phosphate 125 mM pH 7. Après dialyse la solution protéique est centrifugée et l'on obtient 23,5 ml d'une solution à 4,2 mg d'anticorps modifié par ml.

Par dosage spectrophotométrique à 343 nm de la pyridinethione-2 libérée par échange avec le mercapto-2 éthanol, on constate que l'on a obtenu un anticorps portant 3,2 groupements disulfure mixte activé par mole d'anticorps.

c) Réaction de couplage.
A 781 microlitres de la solution d'anticorps activé précédemment obtenue (soit 0,022 micromole), on ajoute 7,3 ml de la solution de chaîne A de ricine modifiée, soit 0,275 micromoles). On laisse le mélange évoluer 18 heures à 30°C. Le milieu réactionnel est ensuite dialysé contre du tampon PBS (phosphate 10 mM, chlorure de sodium 120 mM pH 7,4).

Après centrifugation et examen par électrophorèse en gradient de pollyacrylamide on constate que l'on a obtenu une immunotoxine dont le taux de couplage moyen est pour cette préparation de 0,8 chaîne A NEM méthylée oxydée par mole d'anticorps.

L'immunotoxine à chaîne A de ricine NEM, méthylée, oxydée, obtenue comme indiqué ci-dessus a été étudiée en ce qui concerne ses propriétés pharmacocinétiques et ses propriétés de cytotoxicité spécifique vis-à-vis des cellules cibles.

2) – Propriétés de l'immunotoxine IT [A(NEM)méthylée-La-cystéamine] T 101.

a) Activité de cytotoxicité spécifique.
On constate que cette immunotoxine préparée selon la procédure développée ci-dessus a une très forte activité cytotoxique sur les cellules cibles CEM (CI 50 = 7 × 10$^{-12}$M) établie selon la méthode décrite dans l'exemple 13).

b) Elimination plasmatique.
L'immunotoxine est administrée chez le lapin par injection unique dans une veine de l'oreille (81 microgrammes en chaîne A/kg). Les échantillons de plasma collectés au temps 24 heures sont analysés à l'aide du test immunométrique IRM-3 (exemple 12). Les résultats sont représentés dans le tableau suivant. Les valeurs de 1'IT T101 sont indiquées à titre de comparaison.

| | Concentration plasmatique relative à 22 heures |
|---|---|
| IT [A(NEM)méthylée-La-cystéamine]-T101 | 1,4 % |
| IT T101 | 0,08% |

Vingt deux heures après l'injection, la concentration en IT à chaîne A modifiée est 17,5 fois supérieure à ce qu'elle est avec l'IT T101.

Exemple 17:

Toxicité de la chaîne A à longue durée d'action injectée chez la souris.

Il était important de vérifier quel était l'impact toxicologique global sur l'animal entier de la chaîne A oxydée (la toxicité des immunotoxines étant du même ordre de grandeur que celle de la chaîne A à doses molaires égales). Pour cela, nous avons déterminé la dose léthale 50% de la chaîne A oxydée, administrée par voie intra-veineuse chez la souris Charles River France CDI en comparaison avec celle de la chaîne A native.

Les valeurs trouvées sont indiquées dans le tableau suivant.

| | DL 50 (microgrammes/souris) |
|---|---|
| chaîne A native | 550 |
| chaîne A oxydée | 800 |

Ces résultats montrent que la toxicité de la chaîne A oxydée est inférieure à celle de la chaîne A native. Ceci signifie qu'en dépit d'un accroîssement considérable du taux plasmatique de la chaîne A lorsque celle-ci a été modifiée par oxydation la toxicité du produit n'est non seulement pas accrue, mais qu'au contraire elle est sensiblement diminuée.

On peut donc utiliser en tant que médicament en thérapeutique humaine les immunotoxines à sous-unités cytotoxiques modifiées. Ces immunotoxines modifiées peuvent être utilisées pour le traitement des affections cancéreuses ou non dont les cellules cibles seraient reconnues par l'anticorps utilisé pour la préparation de l'immunotoxine. Les modalités optimales d'administration ainsi que la durée du traitement devront être déterminées dans chaque cas en fonction du sujet et de la nature de l'affection à traiter.

D'une façon plus générale, les glycoprotéines antitumorales dont les motifs glucidiques sont modifiés par oxydation par l'ion periodate et qui ont une demi-vie plus longue que celle des glycoprotéines antitumorales non modifiées correspondantes, sont utiles comme médicaments.

La présente invention concerne donc, selon un aspect ultérieur, des médicaments antitumoraux, dans lesquels une glycoprotéine antitumorale dont les motifs glucidiques sont modifiés par oxydation par l'ion periodate est conditionnée pour être utilisée par voie injectable et de préférence par voie intraveineuse.

## Revendications pour les Etats contractants: BE, CH, DE, FR, GB, IT, LI, LV, NL, SE

1. Glycoprotéine à action antitumorale inactivant les ribosomes, dont les motifs glucidiques sont modifiés par oxydation par l'ion périodate, ladite glycoprotéine étant différente de la ricine entière.

2. Glycoprotéine inactivant les ribosomes, dont les motifs glucidiques sont modifiés par oxydation par l'ion périodate, ayant substantiellement la même activité et une demi-vie plus longue que la glycoprotéine inactivant les ribosomes non modifiée, ladite glycoprotéine étant différente de la ricine entière.

3. Glycoprotéine inactivant les ribosomes et à longue durée d'action, ladite glycoprotéine étant différente de la ricine entière, caractérisée en ce qu'elle est obtenue par traitement d'une solution aqueuse d'une glycoprotéine inactivant les ribosomes, dont les groupes thiol sont éventuellement protégés, avec une solution aqueuse d'un périodate alcalin pendant une période de temps de 0,2 à 24 heures à la température de 0 à 15°C et à l'abri de la lumière, par déblocage éventuel des groupes thiol et par isolement du produit final selon des méthodes connues.

4. Glycoprotéine selon la revendication 3, caractérisée en ce que la solution aqueuse de glycoprotéine est une solution aqueuse de chaîne A de la ricine.

5. Glycoprotéine selon la revendication 4, caractérisée en ce que la chaîne A de la ricine est fonctionalisée, par exemple par méthylation.

6. Glycoprotéine selon la revendication 3, caractérisée en ce que la solution aqueuse de glycoprotéine est une solution aqueuse de gélonine.

7. Glycoprotéine selon la revendication 3, caractérisée en ce que la solution aqueuse de glycoprotéine est une solution aqueuse GPIR MOM.

8. Glycoprotéine selon la revendication 3, caractérisée en ce que la solution aqueuse de glycoprotéine est une solution aqueuse de GPIR Dianthin 30.

9. Glycoprotéine selon la revendication 3, caractérisée en ce que la solution aqueuse de glycoprotéine est une solution aqueuse Dianthin 32. Agrostin A, Agrostin B, Agrostin C. HCl ou d'Asparagus officinalis inhibitor.

10. Glycoprotéine selon la revendication 4, caractérisée en ce qu'elle est obtenue à partir soit d'une chaîne A de ricine qui est la chaîne A de la ricine native ou un fragment de chaîne A de ricine native, soit d'une chaîne A de ricine ou d'un fragment de celle-ci produits par voie de biosynthèse par une cellule dont le patrimoine génétique a été opportunément modifié.

11. Glycoprotéine selon l'une des revendications 4 ou 9, caractérisée en ce qu'elle est obtenue par traitement d'une solution aqueuse de chaîne A de ricine, dont au moins l'un des groupes thiol est protégé par réaction avec le dinitro-2,2' dithio-5,5' dibenzoate, avec une solution aqueuse de périodate de sodium pendant une période de temps de 0,2 à 24 heures, à la température d'environ 4°C et à l'abri de la lumière, par traitement du mélange avec du mercapto-2 éthanol et isolement du produit ainsi obtenu selon des méthodes connues.

12. Glycoprotéine selon la revendication 6, caractérisée en ce qu'elle est obtenue par traitement d'une solution aqueuse de gélonine avec une solution aqueuse de périodate de sodium pendant une période de temps de 0,2 à 24 heures à la température d'environ 4°C, et à l'abri de la lumière, et isolement du produit ainsi obtenu selon des méthodes connues.

13. Procédé pour la préparation d'une glycoprotéine antitumorale selon la revendication 1, caractérisé en ce qu'on soumet la glycoprotéine antitumorale non modifiée à une oxydation par les ions périodate.

14. Procédé pour la préparation d'une glycoprotéine inactivant les ribosomes, et à longue durée d'action, ladite glycoprotéine étant différente de la ricine entière, caractérisé en ce que l'on traite une solution aqueuse d'une glycoprotéine inactivant les ribosomes, dont les groupes thiol sont éventuellement protégés, avec une solution aqueuse d'un périodate alcalin pendant une période de temps de 0,2 à 24 heures, à la température de 0 à 15°C et à l'abri de la lumière, on débloque éventuellement le groupe thiol et on isole le produit final selon les méthodes connues.

15. Procédé selon la revendication 14, caractérisé en ce que, comme produit de départ, on utilise soit une chaîne A de ricine qui est la chaîne A de ricine native ou un fragment de chaîne A de la ricine native, soit une chaîne A de ricine ou un fragment de celle-ci produits par voie de biosynthèse par une cellule dont le patrimoine génétique a été opportunément modifié.

**Revendications pour l'Etat contractant: AT**

1. Procédé pour l'obtention d'une glycoprotéine antitumorale dont les motifs glucidiques sont modifiés, ladite glycoprotéine étant différente de la ricine entière, caractérisé en ce qu'il consiste à soumettre la glycoprotéine antitumorale non modifiée à une oxydation par les ions périodate.

2. Procédé pour l'obtention d'une glycoprotéine inactivant les ribosomes et à longue durée d'action, ladite glycoprotéine étant différente de la ricine entière, caractérisé en ce que l'on traite une solution aqueuse d'une glycoprotéine inactivant les ribosomes, dont les groupes thiol sont éventuellement protégés, avec une solution aqueuse d'un périodate alcalin pendant une période de temps de 0,2 à 24 heures à la température de 0 à 15°C et à l'abri de la lumière, par déblocage éventuel du groupe thiol et par isolement du produit final selon les méthodes connues.

3. Procédé selon la revendication 2, caractérisé en ce que, comme glycoprotéine de départ, on utilise la chaîne A de la ricine, la gélonine, la GPIR MOM, la GPIR Dianthin 30.

4. Procédé selon la revendication 2, caractérisé en ce que, comme glycoprotéine de départ, on utilise l'une des glycoprotéines ci-après: Dianthin 32, Agrostin A, Agrostin B, Agrostin C, HCl, Asparagus officinalis inhibitor.

5. Procédé selon l'une des revendications 2 à 4, caractérisé en ce que comme produit de départ, on utilise soit une chaîne A de ricine qui est la chaîne A de ricine native ou un fragment de chaîne A de la ricine native, soit une chaîne A de ricine ou un fragment de celle-ci produits par voie de biosynthèse par une cellule dont le patrimoine génétique a été opportunément modifié.

6. Procédé selon la revendication 5, caractérisé en ce que, comme produit de départ, on utilise la chaîne A de ricine fonctionnalisée, par exemple par méthylation.

7. Procédé selon l'une des revendications 2 ou 3, caractérisé en ce que l'on traite une solution aqueuse de chaîne A de ricine, dont au moins l'un des groupes thiol est protégé par réaction avec le dinitro-2,2' dithio-5,5' dibenzoate, avec une solution aqueuse de périodate de sodium pendant une période de temps de 0,2 à 24 heures, à la température d'environ 4°C et à l'abri de la lumière, on traite le mélange avec du mercapto-2 éthanol et on isole le produit ainsi obtenu selon des méthodes connues.

8. Procédé selon l'une des revendications 2 ou 3, caractérisé en ce que l'on traite une solution aqueuse de gélonine avec une solution aqueuse de périodate de sodium pendant une période de temps de 0,2 à 24 heures à la température d'environ 4°C, et à l'abri de la lumière, et on isole le produit ainsi obtenu selon des méthodes connues.

**Patentansprüche für die Vertragsstaaten: BE, CH, DE, FR, GB, IT, LU, NL, SE**

1. Glykoprotein mit antitumoraler, Ribosomen inaktivierender Wirkung, dessen Kohlenhydrateinheiten durch Oxidation mit dem Perjodation modifiziert sind, welches Glykoprotein sich vom Vollricin unterscheidet.

2. Ribosomen inaktivierendes Glykoprotein [GPIR], dessen Kohlenhydrateinheiten durch Oxidation mit dem Perjodation modifiziert sind, welches im wesentlichen die gleiche Aktivität und eine längere Halbwertzeit als das nicht modifizierte Ribosomen inaktivierende Glykoprotein hat, welches Glykoprotein sich vom Vollricin unterscheidet.

3. Ribosomen inaktivierendes Glykoprotein mit langer Wirkungsdauer, welches Glykoprotein sich vom Vollricin unterscheidet, dadurch gekennzeichnet, dass es erhalten ist durch Behandlung einer wässerigen Lösung eines Ribosomen inaktivierenden Glykoproteins, dessen Thiolgruppen gegebenenfalls geschützt sind, mit einer wässerigen Lösung eines alkalischen Perjodats während eines Zeitraums von 0,2 bis 24 Stunden bei einer Temperatur von 0 bis 15°C und vor Licht geschützt durch eventuelle Entblockung der Thiolgruppen und durch Isolieren des Endprodukts nach bekannten Methoden.

4. Glykoprotein nach Anspruch 3, dadurch gekennzeichnet, dass die wässerige Glykoproteinlösung eine wässerige Lösung der Kette A von Ricin ist.

5. Glykoprotein nach Anspruch 4, dadurch gekennzeichnet, dass die Kette A des Ricins, beispielsweise durch Methylierung, funktionalisiert ist.

6. Glykoprotein nach Anspruch 3, dadurch gekennzeichnet, dass die wässerige Glykoproteinlösung eine wässerige Geloninlösung ist.

7. Glykoprotein nach Anspruch 3, dadurch gekennzeichnet, dass die wässerige Glykoproteinlösung eine wässerige GPIR-MOM-Lösung ist.

8. Glykoprotein nach Anspruch 3, dadurch gekennzeichnet, dass die wässerige Glykoproteinlösung eine wässerige Lösung von GPIR-Dianthin 30 ist.

9. Glykoprotein nach Anspruch 3, dadurch gekennzeichnet, dass die wässerige Glykoproteinlösung eine wässerige Lösung von Dianthin 32, Agrostin A, Agrostin B, Agrostin C, HCl oder Asparagus officinalis-Inhibitor ist.

10. Glykoprotein nach Anspruch 4, dadurch gekennzeichnet, dass es erhalten ist entweder aus einer Kette A von Ricin, nämlich der nativen Kette A von Ricin oder einem Fragment der nativen Kette A von Ricin, oder aus einer Kette A von Ricin oder eines Fragments desselben, erzeugt auf biosynthetischem Weg mittels einer Zelle, deren genetisches Erbgut entsprechend modifiziert worden ist.

11. Glykoprotein nach einem der Ansprüche 4 oder 9, dadurch gekennzeichnet, dass es erhalten ist durch Behandlung einer wässerigen Lösung der Kette A von Ricin, bei der zumindest eine der Thiolgruppen durch Umsetzung mit 2,2'-Dinitro-5,5'-dithiodibenzoat geschützt wird, mit einer wässerigen Natriumperjodatlösung während eines Zeitraums von 0,2 bis 24 Stunden bei einer Temperatur von etwa 4°C und vor Licht geschützt, durch Behandlung der Mischung mit 2-Mercaptoethanol und durch Isolieren des so erhaltenen Produktes nach bekannten Methoden.

12. Glykoprotein nach Anspruch 6, dadurch gekennzeichnet, dass es erhalten ist durch Behandlung einer wässerigen Geloninlösung mit einer wässerigen Natriumperjodatlösung während eines Zeitraums von 0,2 bis 24 Stunden bei einer Temperatur von etwa 4°C und vor Licht geschützt und Isolieren des so erhaltenen Produkts nach bekannten Methoden.

13. Verfahren zur Herstellung eines antitumoralen Glykoproteins nach Anspruch 1, dadurch gekennzeichnet, dass man das nicht modifizierte antitumorale Glykoprotein einer Oxidation mit Perjodat-Ionen unterzieht.

14. Verfahren zur Herstellung eines Ribosomen inaktivierenden Glykoproteins mit langer Wirkungsdauer, welches Glykoprotein sich vom Vollricin unterscheidet, dadurch gekennzeichnet, dass man eine wässerige Ribosomen inaktivierende Glykoproteinlösung, deren Thiolgruppen gegebenenfalls geschützt sind, mit einer wässerigen Lösung eines alkalischen Perjodats während eines Zeitraums von 0,2 bis 24 Stunden bei einer Temperatur von 0 bis 15°C und vor Licht geschützt

durch eventuelles Entblocken der Thiolgruppe und durch Isolieren des Endprodukts nach bekannten Methoden behandelt.

15. Verfahren nach Anspruch 14, dadurch gekennzeichnet, dass man als Ausgangsprodukt entweder eine Kette A von Ricin, nämlich die native Kette A von Ricin oder ein Fragment der nativen Kette A von Ricin, oder eine Kette A von Ricin oder ein Fragment derselben, hergestellt auf biosynthetischem Weg mittels einer Zelle, deren genetisches Erbgut entsprechend modifiziert worden ist, verwendet.

**Patentansprüche für den Vertragsstaat: AT**

1. Verfahren zur Herstellung eines antitumoralen Glykoproteins, dessen Kohlenhydrateinheiten modifiziert sind, welches Glykoprotein sich vom Vollricin unterscheidet, dadurch gekennzeichnet, dass es darin besteht, dass das nicht modifizierte antitumorale Glykoprotein einer Oxidation mit Perjodationen unterzogen wird.

2. Verfahren zur Herstellung eines Ribosomen inaktivierenden Glykoproteins [GPIR] mit langer Wirkungsdauer, welches Glykoprotein sich vom Vollricin unterscheidet, dadurch gekennzeichnet, dass man eine wässerige Ribosomen inaktivierende Glykoproteinlösung, deren Thiolgruppen gegebenenfalls geschützt sind, mit einer wässerigen Lösung eines alkalischen Perjodats während eines Zeitraums von 0,2 bis 24 Stunden bei einer Temperatur von 0 bis 15°C und vor Licht geschützt, durch eventuelles Entblocken der Thiolgruppe und durch Isolieren des Endprodukts nach bekannten Methoden behandelt.

3. Verfahren nach Anspruch 2, dadurch gekennzeichnet, dass man als Ausgangsglykoprotein die Kette A von Ricin, Gelonin, GPIR-MOM, GPIR-Dianthin 30 verwendet.

4. Verfahren nach Anspruch 2, dadurch gekennzeichnet, dass man als Ausgangsglykoprotein eines der nachstehenden Glykoproteine verwendet: Dianthin 32, Agrostin A, Agrostin B, Agrostin C, HCl, Asparagus offinicalis-Inhibitor.

5. Verfahren nach einem der Ansprüche 2 bis 4, dadurch gekennzeichnet, dass man als Ausgangsprodukt entweder eine Kette A von Ricin, nämlich die native Kette A von Ricin oder ein Fragment der nativen Kette A von Ricin, oder eine Kette A von Ricin oder ein Fragment derselben, hergestellt auf biosynthetischem Weg mittels einer Zelle, deren genetisches Erbgut entsprechend modifiziert worden ist, verwendet.

6. Verfahren nach Anspruch 5, dadurch gekennzeichnet, dass man als Ausgangsprodukt die Kette A von Ricin funktionalisiert, beispielsweise durch Methylierung, verwendet.

7. Verfahren nach einem der Ansprüche 2 oder 3, dadurch gekennzeichnet, dass man eine wässerige Lösung der Kette A von Ricin, bei der zumindest eine der Thiolgruppen durch Umsetzung mit 2,2'-Dinitro-5,5'-dithiodibenzoat geschützt wird, mit einer wässerigen Natriumperjodatlösung während eines Zeitraums von 0,2 bis 24 Stunden bei einer Temperatur von etwa 4°C und vor Licht geschützt durch Behandlung der Mischung mit 2-Mercaptoethanol und durch Isolieren des so erhaltenen Produkts nach bekannten Methoden behandelt.

8. Verfahren nach einem der Ansprüche 2 oder 3, dadurch gekennzeichnet, dass man eine wässerige Geloninlösung mit einer wässerigen Natriumperjodatlösung während eines Zeitraums von 0,2 bis 24 Stunden bei einer Temperatur von etwa 4°C und vor Licht geschützt und Isolieren des so erhaltenen Produkts nach bekannten Methoden behandelt.

### Claims for the contracting States: BE, CH, DE, FR, GB, IT, LI, LU, NL, SE

1. Antitumoral glycoprotein which inactives ribosomes, whose carbohydrate units are modified by oxidation with the periodate ion, said glycoprotein differing from the whole ricin.

2. Glycoprotein which inactivates ribosomes, whose carbohydrate units are modified by oxidation with the periodate ion and which has substantially the same activity as and a longer half-life than the unmodified glycoprotein which inactivates ribosomes, said glycoprotein differing from the whole ricin.

3. Glycoprotein which inactivates ribosomes and has a prolonged action, said glycoprotein differing from the whole ricin, characterized in that it is obtained by treatment of an aqueous solution of a glycoprotein which inactivates ribosomes, the thiol groups of which are optinally protected, with an aqueous solution of an alkali metal periodate, for a period of 0.2 to 24 hours, at a temperature of 0 to 15°C and in the absence of light, unblocking of the thiol groups, if appropriate, and isolation of the final product by known methods.

4. Glycoprotein according to claim 3, characterized in that the aquous solution of glycoprotein is an aqueous solution of A chain of ricin.

5. Glycoprotein according to claim 4, characterized in that the A chain of ricin is functionalized, for example by methylation.

6. Glycoprotein according to claim 3, characterized in that the aqueous solution of glycoprotein is an aqueous solution of gelonine.

7. Glycoprotein according to claim 3, characterized in that the aqueous solution of glycoprotein is an aqueous solution of GPIR MOM.

8. Glycoprotein according to claim 3, characterized in that the aqueous solution of glycoprotein is an aqueous solution of GPIR Dianthin 30.

9. Glycoprotein according to claim 3, characterized in that the aqueous solution of glycoprotein is an aqueous solution of Dianthin 32, Agrostin A, Agrostin B, Agrostin C, HCl or Asparagus officinalis inhibitor.

10. Glycoprotein according to claim 4, characterized in that it is obtained either from an A chain of ricin which is the A chain of native ricin or a fragment of A chain of native ricin, or from an A chain of ricin or a fragment thereof produced biosynthetically by a cell whose genotype has been appropriately modified.

11. Glycoprotein according to one of claims 4 or 9, characterized in that it is obtained by treatment of an aqueous solution of A chain of ricin, at least one of the thiol groups of which is protected by reaction with 2,2'-dinitro-5,5'-dithiodibenzoate, with an aqueous solution of sodium periodate, for a period of 0.2 to 24 hours, at a temperature of about 4°C and in the absence of light, treatment of the mixture with 2-mercaptoethanol and isolation of the resulting product by known methods.

12. Glycoprotein according to claim 6, characterized in that it is obtained by treatment of an aqueous solution of gelonine with an aqueous solution of sodium periodate, for a period of 0.2 to 24 hours, at a temperature of about 4°C and in the absence of light, and isolation of the resulting product by known methods.

13. Process for the preparation of an antitumoral glycoprotein according to claim 1, characterized in that it comprises subjecting the unmodified antitumoral glycoprotein to oxidation with periodate ions.

14. Process for the preparation of a glycoprotein which inactivates ribosomes and has a prolonged action, said glycoprotein differing from whole ricin, characterized in that it comprises treating an aqueous solution of a glycoprotein which inactivates ribosomes, the thiol groups of which are optionally protected, with an aqueous solution of an alkali metal periodate, for a period of 0.2 to 24 hours, at a temperature of 0 to 15°C and in the absence of light, unblocking of the thiol group, if appropriate, and isolation of the final product by known methods.

15. Process according to claim 14, characterized in that the starting material used is either an A chain of ricin which is the A chain of native ricin or a fragment of A chain of native ricin, or an A chain of ricin or a fragment thereof produced biosynthetically by a cell whose genotype has been appropriately modified.

### Claims for contracting State: AT

1. Process for the obtention of an antitumoral glycoprotein whose carbohydrate units are modified, said glycoprotein differing from the whole ricin, characterized in that it consists in submitting an unmodified antitumoral glycoprotein to an oxydation with periodate ions.

2. Process for the obtention of a glycoprotein which inactivates ribosomes and has a prolonged action, said glycoprotein differing from whole ricin, characterized in that it comprises treating an aqueous solution of a glycoprotein which inactivates ribosomes, the thiol groups of which are optionally protected, with an aqueous solution of an alkali metal periodate, for a period of 0.2 to 24 hours, at a temperature of 0 to 15°C and in the absence of light, unblocking of the thiol group, if appropriate, and isolation of the final product by known methods.

3. Process according to claim 2, characterized in that A chain of ricin, gelonine, GPIR MOM and GPIR Dianthin 30 are used as starting glycoprotein.

4. Process according to claim 2, characterized in that one of the following glycoproteins is used as starting glycoprotein: Dianthin 32, Agrostin A, Agrostin B, Agrostin C, HCl, Asparagus officinalis inhibitor.

5. Process according to any one of claims 2 to 4, characterized in that the starting material used is either an A chain of ricin which is the A chain of native ricin of a fragment of A chain of native ricin, or an A chain of ricin or a fragment thereof produced biosynthetically by a cell whose genotype has been appropriately modified.

6. Process according to claim 6, characterized in that the A chain of ricin functionalized, for example by methylation is used as starting material.

7. Process according to any one of claims 2 or 3, characterized in that an aqueous solution of A chain of ricin, at least one of the thiol groups of which is protected by reaction with 2,2'-dinitro-5,5'-dithiodibenzoate is treated with an aqueous solution of sodium periodate, for a period of 0.2 to 24 hours, at a temperature of about 4°C and in the absence of light, the mixture is treated with 2-mercaptoethanol and the resulting product is isolated by known methods.

8. Process according to any one of claims 2 or 3, characterized in that an aqueous solution of gelonine is treated with an aqueous solution of sodium periodate, for a period of 0.2 to 24 hours, at a temperature of about 4°C and in the absence of light, and the resulting product is isolated by known methods.

Fig.1

Elimination plasmatique de la chaîne A, de la chaîne A oxydée

Fig.2

Influence du temps de traitement au périodate de sodium sur la concentration plasmatique de la chaîne A

Fig.3

Influence du temps de traitement au périodate de sodium sur la concentration plasmatique de la chaîne A et de la chaîne A (NEM) méthylée.

Fig.4

Elimination plasmatique de la gélonine, de la gélonine oxydée

Fig.7

Cinétique d'élimination plasmatique de l'IT 101 et IT (A-1a) T 101

EP 0172045 B1

Fig.5

Elimination plasmatique
de la GPIR MOM et de la
GPIR MOM oxydée

Fig.6

Elimination plasmatique
de la GPIR Dianthin 30 et
de la GPIR Dianthin 30 oxydée

27

Fig.8

Cytotoxicité de l'IT (A-1a) T101 et de (A-1a) sur les cellules CEM mesurée par l'inhibition de la synthèse protéique

Fig.9

Cytotoxicité de l'IT (A-1a) T101 et de l'IT T101 sur les cellules CEM mesurée en test clonogénique

29